# EUROPEAN PATENT SPECIFICATION

(11) **EP 1 831 398 B1**
(45) Date of publication and mention of the grant of the patent: **29.10.2014**
(21) Application number: 05852717.7
(22) Date of filing: 01.12.2005
(51) Int. Cl.: G01N 33/574, C12Q 1/68

(54) **Method for determining resistance or sensitivity to taxoid-based medicaments**
Verfahren zur Bestimmung der Resistenz oder Empfindlichkeit gegenüber Taxoiden
Procédé de la détermination de la résistance or de la sensibilité aux taxoïdes

(30) Priority: 08.12.2004 US 634298 P
(43) Date of publication of application: 12.09.2007
(60) Divisional application: 11003911.2
(73) Proprietor: Aventis Pharmaceuticals Inc., Bridgewater, NJ 08807 (US)
(72) Inventor: GRUENEBERG, Dorre, Newtonville, Massachusetts 02460 (US); HUANG, Xi, Arlington, Massachusetts 02474 (US); NATESAN, Sridaran, Ashland, Massachusetts 01721 (US); AUGUST, Paul, Boxford, Massachusetts 01921 (US)
(74) Representative: Zwicker, Jörk
(86) International application number: PCT/US2005/043578
(87) International publication number: WO 2006/062811

(56) References cited:
- WO-A-2004/024064
- WO-A2-2005/020794
- US-B1- 6 410 312
- SUDO TAMOTSU ET AL: "Dependence of paclitaxel sensitivity on a functional spindle assembly checkpoint" CANCER RESEARCH, vol. 64, no. 7, 1 April 2004 (2004-04-01), pages 2502-2508, XP002384388 ISSN: 0008-5472
- SASSE A ET AL: "Coxsackie-and-adenovirus receptor mRNA expression in human heart failure." THE JOURNAL OF GENE MEDICINE. OCT 2003, vol. 5, no. 10, October 2003 (2003-10), pages 876-882, XP002384538 ISSN: 1099-498X
- SAVLI H ET AL: "Quantification of the FLI1 gene expression by real-time quantitative RT-PCR" TURKISH JOURNAL OF MEDICAL SCIENCES 2003 TURKEY, vol. 33, no. 1, 2003, pages 21-25, XP009067688 ISSN: 1300-0144
- LEE ERIC A ET AL: "Inactivation of the mitotic checkpoint as a determinant of the efficacy of microtubule-targeted drugs in killing human cancer cells" MOLECULAR CANCER THERAPEUTICS, vol. 3, no. 6, June 2004 (2004-06), pages 661-669, XP002384389 ISSN: 1535-7163
- CAHILL D P ET AL: "Mutations of mitotic checkpoint genes in human cancers" NATURE, NATURE PUBLISHING GROUP, LONDON, GB, vol. 392, 19 March 1998 (1998-03-19), pages 300-303, XP002124486 ISSN: 0028-0836
- CHANG JENNY C ET AL: "Gene expression profiling for the prediction of therapeutic response to docetaxel in patients with breast cancer." LANCET. 2 AUG 2003, vol. 362, no. 9381, 2 August 2003 (2003-08-02), pages 362-369, XP002384390 ISSN: 1474-547X
- ZHANG CHRISTINE C ET AL: "DNA damage increases sensitivity to Vinca alkaloids and decreases sensitivity to taxanes through p53-dependent repression of microtubule-associated protein 41" CANCER RESEARCH, vol. 59, no. 15, 1 August 1999 (1999-08-01), pages 3663-3670, XP002384391 ISSN: 0008-5472
- DUAN ZHENFENG ET AL: "Inhibition of ABCB1 (MDR1) and ABCB4 (MDR3) expression by small interfering RNA and reversal of paclitaxel resistance in human ovarian cancer cells" MOLECULAR CANCER THERAPEUTICS, vol. 3, no. 7, July 2004 (2004-07), pages 833-838, XP002384392 ISSN: 1535-7163
- MCGROGAN B T ET AL: "Taxanes, microtubules and chemoresistant breast cancer", BBA - REVIEWS ON CANCER, ELSEVIER SCIENCE BV, AMSTERDAM, NL, vol. 1785, no. 2, 1 April 2008 (2008-04-01), pages 96-132, XP022615363, ISSN: 0304-419X, DOI: 10.1016/J.BBCAN.2007.10.004 [retrieved on 2007-11-12]
- TANAKA K ET AL: "Mitotic Checkpoint Genes, hsMAD2 and BubR1, in Oesophageal Squamous Cancer Cells and their Association with 5-fluorouracil and Cisplatin-based Radiochemotherapy", CLINICAL ONCOLOGY, W.B. SAUNDERS, vol. 20, no. 8, 1 October 2008 (2008-10-01), pages 639-646, XP025407782, ISSN: 0936-6555, DOI: 10.1016/J.CLON.2008.06.010 [retrieved on 2008-08-08]
- FU YUNFENG ET AL: "Weakened spindle checkpoint with reduced BubR1 expression in paclitaxel-resistant ovarian carcinoma cell line SKOV3-TR30.", GYNECOLOGIC ONCOLOGY APR 2007 LNKD- PUBMED:17234259, vol. 105, no. 1, April 2007 (2007-04), pages 66-73, ISSN: 0090-8258
- CHARLES SWANTON ET AL: 'Chromosomal Instability, Colorectal Cancer and Taxane Resistance' CELL CYCLE vol. 5, no. 8, 15 April 2006, pages 818 - 823, XP055043695 DOI: 10.4161/cc.5.8.2682 ISSN: 1538-4101

## Description

### FIELD OF THE INVENTION

The present invention relates to novel, useful and heretofore unknown methods that may predict or monitor a patient's response to a molecule of the taxoid family by measuring the increase or decrease of a specific genetic marker, BubR1, as compared to controls. The present invention also provides kits that predict or monitor patients' response to a molecule of the taxoid family by measuring nucleic acid or protein levels of a particular genetic marker, BubR1, and comparing its levels to controls or reference markers.

### BACKGROUND OF THE INVENTON

Docetaxel is an anti-mitotic drug widely used for the treatment of breast, lung and ovarian cancer, and to a lesser extent used for treating head and neck, gastric and prostate carcinomas (Hong, Oncology 16:9, 2002). Docetaxel inhibits microtubule dynamics by binding to beta-tubulin and blocking disassembly of alpha- and beta-tubulin heterodimers thus abrogating tumor growth (Ringel and Horwitz, J Natl Cancer Inst 83:288,1991). The antitumor activities of docetaxel and a related taxane, paclitaxel arise from targeting microtubules of the mitotic spindle to impede chromosome alignment and segregation, block cell cycle progression and activate apoptosis pathways (Wang et al., Cancer 88:2619, 2000). The proapoptotic activities of paclitaxel have been linked with the phosphorylation and inactivation of Bcl-2 through cell signaling events that involve p53/p21waf1/Cip1, raf/ras and mitogen-activated protein kinases (MAPKs) (Wang et al., Cancer 88:2619, 2000). Although docetaxel is a more potent anti-cancer agent than paclitaxel, the pathways involved in its cytotoxicity are less well defined (Katsumata, Br J Cancer 89:S9,2003). Docetaxel-induced apoptosis has been observed in select cell lines through a mechanism involving Bcl-2 phosphorylation and caspase-3 activation (Kolfschoten et al., Biochem Pharmacol 63:733, 2002). Other proteins that modulate taxane-induced cell killing in different cultured lines include Aurora-A in HeLa cells (Anand et al., Cancer Cell 3:51, 2003), HER-2 in breast cancer cells (Tanabe et al., Int J Oncol 22:875, 2003), p21waf1/Cip1 in glioblastoma cells (Li et al., J Biol Chem 277:11352, 2002) and JNK/MKK1 in ovarian cancer cells (Lee et al., J Biol Chem 273:28253,1998).

Sudo et al. (Cancer Research, 2004, 64(7):2502-2508) discloses that treatment of cancer cell cultures with siRNA against the mitotic checkpoint genes Mad2 and BubR1 leads to paclitaxel resistance. Sudo et al. further teaches that assessing the function of the checkpoint in human cancer samples by analyzing mutations of genes or protein expressions of BubR1 and other components of the checkpoint machinery would be impractical, and suggests that Cdk1 activity should be used as a predictive marker.

There remains a need in the field to identify drugablc proteins that mediate docetaxel resistance, which could lead to their utilization in drug discovery to identify reagents (small molecules, siRNA, etc.) that abrogate their in vivo function and potentially sensitize cells to anti-tumor drug chemotherapy. There also remains a need in the field for an assay which could confidently predict in advance of chemotherapy treatment which patients would and would not respond to anti-tumor docetaxel drug based chemotherapy. Applicants describe herein a novel assay that can predict resistance and or sensitivity to docetaxel and provide screening assays for the development of new therapies to abrogate drug resistance in cancer.

### SUMMARY OF THE INVENTION

The scope of the present invention is defined in the appended claims.

In accordance with the present invention, there is provided novel and useful methods to monitor and/or predict a patient's response to a molecule of the taxoid family by comparing the levels of activation and/or expression of a specific genetic marker, BubR1, in patients and controls.

In one embodiment, the present invention provides a method for predicting or monitoring a cancer patient's response to a molecule of the taxoid family, comprising the steps of:
a) obtaining a test sample from a cancerous area of said patient;
b) obtaining a control sample;
c) measuring the level of BubR1; and
d) comparing the measured levels of BubR1 in the test sample and the control sample;
wherein a decrease in the level of BubR1 measured in the test sample as compared to the control sample indicates an increased resistance to a molecule of the taxoid family.

The genetic markers disclosed are BubR1, Homo sapiens similar to protein kinase (BUBR1) mRNA, complete cds (GenBank accession number: AF046079). Also disclosed are:
Mad2, Homo sapiens mRNA for MAD2 protein (GenBank accession number: AJ000186);
Mps1, Homo sapiens TTK protein kinase (TTK), mRNA (GenBank accession number: NM_003318);
GEFT for Rac1/CDC42, Homo sapiens RAC/CDC42 exchange factor (GEFT), transcript variant 2, mRNA (GenBank accession number: NM_133483);
Bub1, Homo sapiens BUB 1 budding uninhibited by benzimidazoles 1 homolog (yeast) (BUB1), mRNA (GenBank accession number: NM_004336);
hSepharase, Homo sapiens extra spindle poles like 1 (S. cerevisiae) (ESPL1), mRNA (GenBank accession number: NM_012291);
CamKIId, Homo sapiens calcium/calmodulin-dependent protein kinase (CaM kinase) II delta (CAMK2D), transcript variant 3, mRNA (GenBank accession number: NM_001221);
CDK6, Homo sapiens cyclin-dependent kinase 6 (CDK6), mRNA (GenBank accession number: NM_001259); and
GRB2, Homo sapiens growth factor receptor-bound protein 2 (GRB2), transcript variant 1, mRNA (GenBank accession number: NM_002086).

Disclosed is a method for predicting or monitoring a cancer patient's response to a molecule of the taxoid family, comprising the steps of:
a) obtaining a test sample from a cancerous area of said patient;
b) obtaining a control sample;
c) measuring the level of one or more genetic markers; and
d) comparing the measured levels of said one or more genetic markers in the test sample and the control sample;
wherein a decrease in the level of said one or more genetic markers measured in the test sample as compared to the control sample indicates an increased sensitivity to a molecule of the taxoid family.

In this particular method one or more genetic markers may be selected from the group consisting of:
P21(Waf1), Homo sapiens cyclin-dependent kinase inhibitor 1A (p21, Cip1) (CDKN1A), transcript variant 1, mRNA (GenBank accession number: NM_000389);
Pim-1, Homo sapiens pim-1 oncogene (PIM1), mRNA (GenBank accession number: NM_002648);
GBP-1, Homo sapiens guanylate binding protein 1, interferon-inducible, 67kDa (GBP1), mRNA (GenBank accession number: NM_002053);
RXRA, Homo sapiens retinoid X receptor, alpha (RXRA), mRNA (GenBank accession number: NM_002957);
SPF45, Homo sapiens RNA binding motif protein 17 (RBM17), mRNA (GenBank accession number: NM_032905);
Heel, Homo sapiens kinetochore associated 2 (KNTC2), mRNA (GenBank accession number: M_006101);
Raf1, Human mRNA for raf oncogene (GenBank accession number: X03484);
Aurora A, Homo sapiens aurora-related kinase 1 (ARK1) mRNA, complete cds (GenBank accession number: AF008551);
TACC3, Homo sapiens transforming, acidic coiled-coil containing protein 3 (TACC3), mRNA (GenBank accession number: NM_006342);
RelB, Homo sapiens v-rel reticuloendotheliosis viral oncogene homolog B, nuclear factor of kappa light polypeptide gene enhancer in B-cells 3 (avian) (RELB), mRNA (GenBank accession number: NM_006509);
PRKCD, Homo sapiens protein kinase C, delta (PRKCD), transcript variant 1, mRNA (GenBank accession number: NM_006254);
BRAF35, Homo sapiens high-mobility group 20B (HMG20B), mRNA (GenBank accession number: NM_006339);
HSPA1L, Homo sapiens heat shock 70kDa protein 1A (HSPA1A), mRNA (GenBank accession number: NM_005345);
STK11, Homo sapiens serine/threonine kinase 11 (Peutz-Jeghers syndrome) (STK11), mRNA (GenBank accession number: NM_000455); and
MKK3, Homo sapiens MAP kinase kinase 3 (MKK3) mRNA, complete cds (GenBank accession number: L36719).

In yet a further embodiment, the present invention relates to a method for predicting or monitoring a cancer patient's response to a molecule of the taxoid family, comprising the steps of:
a) obtaining a test sample from a cancerous area of said patient;
b) measuring the level of the genetic marker:
   BubR1, Homo sapiens similar to protein kinase (BUBR1) mRNA, complete cds (GenBank accession number: AF046079);
c) measuring the level of one or more reference genetic markers selected from the group consisting of:
   GAPDH, Homo sapiens glyceraldehyde-3-phosphate dehydrogenase (GAPD), mRNA (GenBank accession number: NM_002046); and
   RPS9, Homo sapiens cDNA clone IMAGE:6647283, partial cds (GenBank accession number: BC071941);
d) comparing the measured levels of BubR1 and said one or more reference genetic markers in the test sample;
wherein a decrease in the level of BubR1 as compared to the level of said one or more reference genetic markers indicates an increased resistance to a molecule of the taxoid family.

Also disclosed is a method for predicting or monitoring a cancer patient's response to a molecule of the taxoid family, comprising the steps of:
a) obtaining a test sample from a cancerous area of said patient;
b) measuring the level of one or more genetic markers selected from the group consisting of:
   P21(Waf1), Homo sapiens cyclin-dependent kinase inhibitor 1A (p21, Cip1) (CDKN1A), transcript variant 1, mRNA (GenBank accession number: NM_000389);
   Pim-1, Homo sapiens pim-1 oncogene (PIM1), mRNA (GenBank accession number: NM_002648);
   GBP-1, Homo sapiens guanylate binding protein 1, interferon-inducible, 67kDa (GBP1), mRNA (GenBank accession number: NM_002053);
   RXRA, Homo sapiens retinoid X receptor, alpha (RXRA), mRNA (GenBank accession number: NM_002957);
   SPF45, Homo sapiens RNA binding motif protein 17 (RBM17), mRNA (GenBank accession number: NM_032905);
   Heel, Homo sapiens kinetochore associated 2 (KNTC2), mRNA (GenBank accession number: NM_006101);
   Raf1, Human mRNA for raf oncogene (GenBank accession number: X03484);
   Aurora A, Homo sapiens aurora-related kinase 1 (ARK1) mRNA, complete cds (GenBank accession number: AF008551);
   TACC3, Homo sapiens transforming, acidic coiled-coil containing protein 3 (TACC3), mRNA (GenBank accession number: NM_006342);
   RelB, Homo sapiens v-rel reticuloendotheliosis viral oncogene homolog B, nuclear factor of kappa light polypeptide gene enhancer in B-cells 3 (avian) (RELB), mRNA (GenBank accession number: NM_006509);
   PRKCD, Homo sapiens protein kinase C, delta (PRKCD), transcript variant 1, mRNA (GenBank accession number: NM_006254);
   BRAF35, Homo sapiens high-mobility group 20B (HMG20B), mRNA (GenBank accession number: NM_006339);
   HSPA1L, Homo sapiens heat shock 70kDa protein 1A (HSPA1A), mRNA (GenBank accession number: NM_005345);
   STK11, Homo sapiens serine/threonine kinase 11 (Peutz-Jeghers syndrome) (STK11), mRNA (GenBank accession number: NM_000455); and
   MKK3, Homo sapiens MAP kinase kinase 3 (MKK3) mRNA, complete cds (GenBank accession number: L36719);
c) measuring the level of one or more reference genetic markers selected from the group consisting of
   GAPDH, Homo sapiens glyceraldehyde-3-phosphate dehydrogenase (GAPD), mRNA (GenBank accession number: NM_002046); and
   RPS9, Homo sapiens cDNA clone IMAGE:6647283, partial cds (GenBank accession number: BC071941);
d) comparing the measured levels of said one or more genetic markers and said one or more reference genetic markers in the test sample;
   wherein a decrease in the level of said one or more genetic markers as compared to the level of said one or more reference genetic markers indicates an increased susceptibility to a molecule of the taxoid family.

The present invention relates to methods for predicting or monitoring a cancer patient's response to a molecule of the taxoid family as defined in the appended claims. This, a further embodiment of the present inventions includes the taxoid family molecules paclitaxel, docetaxel XRP9881 and XRP6258

BubR1 may be evaluated by measuring the levels of RNA or protein using a variety of techniques that are described in further detail below. Other embodiments of the present invention relate to the use of kits as defined in the claims for predicting or monitoring a patient's response to a molecule of the taxoid family.

The above aspects and other aspects, features, and advantages of the present invention will be better understood from the following detailed description taken in conjunction with the accompanying figures.

### BRIEF DESCRIPTION OF THE DRAWINGS

Figure 1 shows the characterization of genes whose downregulation confer docetaxel resistance. Docetaxel generated dose response curves shown for HCT116 cells transfected with RB1, BubR1, Mad2 and Mps1 siRNAs (solid squares) compared to control siRNA (open squares). RB1 was an example of a siRNA that did not score in the screen. Cell viability was measured at an absorbance of 450 nM following WST-1 addition. The minimum ratio (MR) is the WST-1 value for a gene over the control at 40 nM docetaxel.
Figure 2 shows dose response curves of BubR1 and Mps1 shRNAs (solid squares) compared to control shRNA (open squares).
Figure 3 shows TaqMan real-time PCR analysis of BubR1 and Mps1 mRNA levels in cells containing BubR1 and Mps1 shRNA, respectively.
Figure 4 shows cell viability and morphology shown for HCT116 cells transfected with Mad2, BubR1 and Mps1 siRNAs with or without docetaxel treatment. Twenty-four hours after transfection, cells were left untreated (-Docetaxel) or treated with 200 nM docetaxel (+Docetaxel) and stained with Calcein-AM to visualize live cells 16 and 72 hours after treatment.
Figure 5 shows mitotic indices for HCT116 cells transfected with Mad2, BubR1, Mps1 and control siRNAs following docetaxel treatment. Twenty-four hours after transfection cells were treated with 200 nM docetaxel for 16 and 72 hours and processed. Mitotic cells were detected by a phosphorylated histone H3 antibody, shown here as red punctuate staining over the nucleus of cells (Mitotic Index Kit, Cellomics). Nuclei were stained blue with Hoechst dye.
Figure 6 shows TaqMan real-time PCR analysis of Mps1, BubR1 and Mad2 mRNA levels in HCT116 cells transfected with Mps1, BubR1 and Mad2 siRNA, respectively.
Figure 7 shows cell cycle analysis of HCT116 cells transfected with three mitotic checkpoint gene siRNAs. After transfection with Mps1, BubR1 and Mad2 siRNAs, cells were untreated (-Docetaxel) or treated with 200 nM docetaxel (+Docetaxel) for 24 hours. Seventy-two hours after the addition of docetaxel, cells were harvested and analyzed for cell cycle. Numbers above peaks indicate DNA content such as 2N, 4N, 8N, 16N or 32N.
Figure 8 shows clonogenic cell survival assay using stable knockdown cell lines. HCT116 cells containing BubR1 or vector control shRNAs were plated on 10 cm dishes and maintained in 5 nM docetaxel for 10 days. Colonies were washed with PBS and stained with crystal violet.
Figure 9 shows several dose response curves of genes whose down-regulation confer increased sensitivity to docetaxel. Doectaxel generated dose response curves shown for HCT116 cells transfected with RB1, Pim-1, p21, Aurora A and TACC3 siRNAs (solid squares) compared to control siRNA (open squares). The minimum ration (MR) is the ratio of WST-1 readout of a gene over that of the control at 40 nM docetaxel concentration. The IC50 (IR) ratio is the ratio of IC50 of a gene over that of the control.
Figure 10 shows a dose response curves of Aurora A shRNA (solid squares) compared to vector control (open squares).
Figure 11 shows TaqMan real-time PCR analysis of Aurora A mRNA levels in cells containing Aurora A shRNA.
Figure 12 shows differences in proliferation of HCT116 cells transfected with Pim-1 and TACC3 siRNAs (solid squares) compared to control siRNA (open squares). Number of cells remaining in 6-well plates at different time points after transfection with Pim-1, TACC3 and control siRNAs.
Figure 13 shows TaqMan analysis of Pim-1 and TACC3 mRNA levels after siRNA transfection.
Figure 14 shows active caspase-3 levels in HCT116 cells transfected with Pim-1 and BubR1 siRNAs. Active caspase-3 levels were shown as fluorescent intensity using the active caspase-3 beadmates kit (Upstate). Three docetaxel concentrations, 0, 5 and 40 nM, and three time points, 24, 48 and 72 hours after docetaxel addition were examined.
Figure 15 shows AKT phosphorylation levels in HCT116 cells transfected with Pim-1 and BubR1 siRNAs. The ratio of phosphorylated and total AKT levels was determined using a bead-based assay (Biosource). Twenty-four hours after transfection cells were left untreated or treated with 5 and 40 nM docetaxel. Forty-eight hours after docetaxel treatment cells lysates were analyzed. The phosphorylation level was calculated as the ratio of phosphorylated AKT (p-AKT) over total AKT (t-AKT).
Figure 16 shows Western blots showing the reduced levels of Pim-1 and BubR1 48 hours after transfection.

### DETAILED DESCRIPTION OF THE INVENTION

The present disclosure is broadly based upon applicants' identification of gene markers that can predict whether a test subject is resistant or sensitive to drugs in the taxoid family. Using a cell based RNA interference (RNAi) screen, applicants observed that drug resistance or sensitivity to docetaxel was associated with particular gene markers.

It is noted that numerous terms and phases are used throughout the instant specification and claims. Definitions of these terms and phrases are provided below:
As used herein, the term "prognosis" refers to the prediction or the likelihood of cancer attributable death or progression, including drug resistance as well as recurrence, metastatic spread, and neoplastic disease.

As used herein, the term "prediction" refers to the likelihood that a patient will respond either favorably or unfavorably to a drug or set of drugs, and also the extent of those responses Such as whether the patient will survive, following surgical removal of the primary tumor and/or chemotherapy for a certain period of time without cancer recurrence. Predictive methods envisioned by the present invention can be used clinically to make treatment decisions by choosing the most appropriate treatment modalities, in particular a chemotherapeutic of the taxoid family, for any particular patient. The predictive methods of the present invention are valuable tools in predicting if a patient is likely to respond favorably to a taxoid-based treatment.

As used herein, the term "tumor," refers to all neoplastic cell growth and proliferation, whether malignant or benign, and all pre-cancerous and cancerous cells and tissues.

As used herein, the term "cancer" and "cancerous" refers to a physiological condition in mammals typically characterized by unregulated cell growth. Examples of cancer include but are not limited to the following: breast cancer; colon cancer; lung cancer; prostate cancer; hepatocellular cancer; gastric cancer; pancreatic cancer; cervical and ovarian cancer; liver cancer; bladder cancer; cancer of the urinary tract; carcinoma; melanoma; brain cancer including glioblastomas and medulloblastomas; biliary tract cancer; choriocarcinoma; esophageal cancer; gastric cancer; hematological neoplasms including acute lymphocytic and myelogenous leukemia; multiple myeloma; AIDS-associated leukemia and adult T-cell leukemia lymphoma; intraepithelial neoplasms including Bowen's disease and Paget's disease; lymphomas including Hodgkin's disease and lymnphocytic lymphomas; neuroblastomas; oral cancer including squamous cell carcinoma, sarcomas including leiomyosarcoma, rhabdomyosarcoma, liposarcoma, fibrosarcoma, and osteosarcoma; skin cancer including melanoma, Kaposi's sarcoma, basocellular cancer, and squamous cell cancer; testicular cancer including germinal tumors such as seminoma, non-seminoma (teratomas, choriocarcinomas), stromal tumors, and germ cell tumors; thyroid cancer including thyroid adenocarcinoma and modullar carcinoma; and renal cancer including adenocarcinoma and Wilms tumor..

As used herein, the term "patient" refers preferably to a human but may also include a non-human primate, cow, horse, pig, sheep, goat, dog, cat or rodent. Preferably the subject is a human either suspected of having cancer, or having been diagnosed with cancer or in a high-risk category for developing cancer, for example, having a family history of cancer. In a preferred embodiment of the invention the cancer is breast cancer. Methods for identifying subjects suspected of having cancer may include manual examination, biopsy, subject's family medical history, subject's medical history, or a number of imaging technologies such as mammography, magnetic resonance imaging, magnetic resonance spectroscopy, or positron emission tomography. Diagnostic methods for cancer and the clinical characterizations of cancer diagnoses are well-known to those of skill in the medical arts.

As used herein, the term "sample" is tissue obtained using methods well-known to those of ordinary skill in the related medical arts. Methods such as biopsy include gross apportioning of a mass, microdissection, laser-based microdissection, or other art-known cell-separation methods. Because of the variability of the cell types in diseased-tissue biopsy material, and the variability in sensitivity of the diagnostic methods used, the sample size required for analysis may range from 1, 10, 50, 100, 200, 300, 500, 1000, 5000, 10,000, to 50,000 or more cells. The appropriate sample size may be determined based on the cellular composition and condition of the biopsy and the standard preparative steps for this determination and subsequent isolation of the nucleic acid for use in the invention are well known to one of ordinary skill in the art. For example, a sample from a biopsy may be sufficient for assessment of RNA expression without amplification. Conversely, the lack of a suitable number of cells in a small biopsy region may require use of RNA conversion and/or amplification methods or other methods to enhance resolution of the nucleic acid molecules. Such methods, which allow use of limited biopsy materials, are well known to those of ordinary skill in the art. Some examples include but are not limited to direct RNA amplification, reverse transcription of RNA to cDNA, amplification of cDNA, or the generation of radio-labeled nucleic acids.

As used herein, the term "test with respect to a sample refers to a sample taken from a cancerous area of the body or from an area of the body exhibiting a stage of or characteristics of cancer.

As used herein, the term "control" with respect to a sample refers to samples which are used for comparative purposes. Preferably, these samples are "control" in the sense that the samples do not exhibit any indication of, or are believed to have, any disease or condition that would affect gene expression, particularly in respect of the disease for which they are to be used as the standard. Alternatively, it may be appreciated that different stages of a disease or condition can be compared and in such cases, the "control" sample corresponds to the earlier stage of the disease or condition. For example, a control sample can be a sample taken from a non-cancerous but comparable area of the body. Additionally, a control sample may be a comparable area of the body from the same subject or a non-cancerous area of the body from a second subject substantially similar to the first subject (same or similar species, age, weight, sex, etc.). Finally, a control sample could also be from a cancerous area of a second subject who respond well to treatment using a molecule of the taxoid family.

A "nucleic acid molecule" refers to the phosphate ester polymeric form of ribonucleosides (adenosine, guanosine, uridine or cytidine; "RNA molecules") or deoxyribonucleosides (deoxyadenosine, deoxyguanosine, deoxythymidine, or deoxycytidine; "DNA molecules"), or any phosphoester analogs thereof, such as phosphorothioates and thioesters, in either single stranded form, or a double-stranded helix. Double stranded DNA-DNA, DNA-RNA and RNA-RNA helices are possible. The term nucleic acid molecule, and in particular DNA or RNA molecule, refers only to the primary and secondary structure of the molecule, and does not limit it to any particular tertiary forms. Thus, this term includes double-stranded DNA found, *inter alia,* in linear or circular DNA molecules (*e.g.,* restriction fragments), plasmids, and chromosomes. In discussing the structure of particular double-stranded DNA molecules, sequences may be described herein according to the normal convention of giving only the sequence in the 5' to 3' direction along the non-transcribed strand of DNA (*i.e.,* the strand having a sequence homologous to the mRNA). A "recombinant DNA molecule" is a DNA molecule that has undergone a molecular biological manipulation.

As used herein, the term "portion" of an isolated nucleic acid molecule that encodes for a particular protein, refers to a part or fragment of the isolated nucleic acid molecule that comprises a sufficient number of contiguous nucleotides that encode for a peptide or polypeptide. Naturally, a "portion" of an isolated nucleic acid molecule is greater than one nucleotide, and the peptide or polypeptide encoded by the portion contains numerous amino acid residues, as described in the definitions of peptide and polypeptide below.

As used herein, the term "peptide" refers to two or more amino acids covalently joined by peptide binds. In a particular embodiment, a peptide comprises at least 10, preferably at least 20, more preferably at least 30, even more preferably at least 40, and most preferably 50 or more amino acids.

As used herein, the term "polypeptide" refers to a linear polymer composed of multiple contiguous amino acids. In particular, a polypeptide may possess a molecular weight greater than 100 kD.

As used herein, the term "genetic marker" refers to a physiological composition whose measured RNA or protein level within a sample serves to predict whether a test subject is resistant or sensitive to drugs in the taxoid family. Moreover, a genetic marker may encode the particular protein or alternatively, may serve as a "surrogate" marker for a protein whose activity is related to the level of the genetic marker in a bodily sample. This relationship may be direct, wherein a decrease in the level of protein activity corresponds to a decrease in the level of the genetic marker, or alternatively, the relationship may be inverse, wherein a decrease in the level of protein activity corresponds to an increase in the level of the genetic marker. The measured levels of a certain gene marker can predict whether a test subject is resistant to drugs in the taxoid family,wherein said gene marker is
BubR1, Homo sapiens similar to protein kinase (BUBR1) mRNA, complete cds (GenBank accession number: AF046079). Also disclosed are
Mad2, Homo sapiens mRNA for MAD2 protein (GenBank accession number: J000186);
Mps1, Homo sapiens TTK protein kinase (TTK), mRNA (GenBank accession number: NM_003318);
GEFT for Rac1/CDC42, Homo sapiens RAC/CDC42 exchange factor (GEFT), transcript variant 2, mRNA (GenBank accession number: NM_133483);
Bub1, Homo sapiens BUB1 budding uninhibited by benzimidazoles 1 homolog (yeast) (BUB1), mRNA (GenBank accession number: NM_004336);
hSepharase, Homo sapiens extra spindle poles like 1 (S. cerevisiae) (ESPL1), mRNA (GenBank accession number: NM_012291);
CamKIId, Homo sapiens calcium/calmodulin-dependent protein kinase (CaM kinase) II delta (CAMK2D), transcript variant 3, mRNA (GenBank accession number: NM_001221);
CDK6, Homo sapiens cyclin-dependent kinase 6 (CDK6), mRNA (GenBank accession number: NM_001259); and
GRB2, Homo sapiens growth factor receptor-bound protein 2 (GRB2), transcript variant 1, mRNA (GenBank accession number: NM_002086)
P21(Waf1), Homo sapiens cyclin-dependent kinase inhibitor 1A (p21, Cip1) (CDKN1A), transcript variant 1, mRNA (GenBank accession number: NM_000389);
Pim-1, Homo sapiens pim-1 oncogene (PIM1), mRNA (GenBank accession number: NM_002648);
GBP-1, Homo sapiens guanylate binding protein 1, interferon-inducible, 67kDa (GBP1), mRNA (GenBank accession number: NM_002053);
RXRA, Homo sapiens retinoid X receptor, alpha (RXRA), mRNA (GenBank accession number: NM_002957);
SPF45, Homo sapiens RNA binding motif protein 17 (RBM17), mRNA (GenBank accession number: NM_032905);
Heel, Homo sapiens kinetochore associated 2 (KNTC2), mRNA (GenBank accession number: NM_006101);
Raf1, Human mRNA for raf oncogene (GenBank accession number: X03484); Aurora A, Homo sapiens aurora-related kinase 1 (ARK1) mRNA, complete cds (GenBank accession number: AF008551);
TACC3, Homo sapiens transforming, acidic coiled-coil containing protein 3 (TACC3), mRNA (GenBank accession number: NM_006342);
RelB, Homo sapiens v-rel reticuloendotheliosis viral oncogene homolog B, nuclear factor of kappa light polypeptide gene enhancer in B-cells 3 (avian) (RELB), mRNA (GenBank accession number: NM_006509);
PRKCD, Homo sapiens protein kinase C, delta (PRKCD), transcript variant 1, mRNA (GenBank accession number: NM_006254);
BRAF35, Homo sapiens high-mobility group 20B (HMG20B), mRNA (GenBank accession number: NM_006339);
HSPA1L, Homo sapiens heat shock 70kDa protein 1A (HSPA1A), mRNA (GenBank accession number: NM_005345);
STK11, Homo sapiens serine/threonine kinase 11 (Peutz-Jeghers syndrome) (STK11), mRNA (GenBank accession number: NM_000455); and
MKK3, Homo sapiens MAP kinase kinase 3 (MKK3) mRNA, complete cds (GenBank accession number: L36719)

As used herein, the term "reference genetic marker" refers to a physiological composition whose measured RNA or protein levels remain unchanged before, during or after exposure to drugs in the taxoid family. "Reference genetic markers" are referred to as housekeeping genes. These are genes that are selected based on the relatively invariable levels of expression in the system which is being examined, for example, the in a particular disease such as cancer. Housekeeping genes are used to normalize results of expression. Examples of such genetic markers include, but certainly are not limited to:
GAPDH, Homo sapiens glyceraldehyde-3-phosphate dehydrogenase (GAPD), mRNA (GenBank accession number: NM_002046); and
RPS9, Homo sapiens cDNA clone IMAGE:6647283, partial cds (GenBank accession number: BC071941).

As used herein, the term "a molecule of the taxoid family" refers to a class of chemotherapeutic compounds to belonging to the taxane family. Specific members of the taxoid family include but are not limited to paclitaxel (taxol), docetaxel (taxotere) and analogs thereof (*i.e*.,XRP9881 and XRP6258; see Ojima and Geney, Curr Opin Investig Drugs 4:737, 2004). Since this class of molecules are beta-tubulin binders and stabilize the polymerized form of the microtubule similar to docetaxel, it is anticipated that the clinical expresson of the biomarker herein described will reflect similar response states to these drugs.

As used herein, the terms "molecule", "compound" or "agent" refer to any composition presently known or subsequently discovered. Examples of compounds or agents having applications herein include organic compounds (e.g., man made, naturally occurring and optically active), peptides (man made, naturally occurring, and optically active, i.e., either D or L amino acids), carbohydrates, nucleic acid molecules, etc.

As used herein, "stringency of hybridization" or 'hybridization under stringent conditions" refers to conditions readily determinable by one of ordinary skill in the art, and generally is an empirical calculation dependent upon probe length, washing temperature, and salt concentration. In general, longer probes require higher temperatures for proper annealing, while shorter probes need lower temperatures. Hybridization generally depends on the ability of denatured DNA to reanneal when complementary strands are present in an environment below their melting temperature. The higher the degree of desired homology between the probe and hybridizable sequence, the higher the relative temperature which can be used. As a result, it follows that higher relative temperatures would tend to make the reaction conditions more stringent, while lower temperatures less so. For additional details and explanation of stringency of hybridization reactions, see Ausubel et al., Current Protocols in Molecular Biology, Wiley Interscience Publishers, (1995).

As used herein, "stringent conditions" or "high stringency conditions", refers to those parameters that: (1) employ low ionic strength and high temperature for washing, for example 0.015 M sodium chloride/0.0015 M sodium citrate/0.1% sodium dodecyl sulfate at 50° C.; (2) employ during hybridization a denaturing agent, such as formamide, for example, 50% (v/v) formamide with 0.1 % bovine serum albumin/0.1% Ficoll/0.1% polyvinylpyrrolidone/50 mM sodium phosphate buffer at pH 6.5 with 750 mM sodium chloride, 75 mM sodium citrate at 42° C.; or (3) overnight hybridization in a solution that employs 50% formamide, 5x SSC (0.75 M NaCl, 0.075 M sodium citrate), 50 mM sodium phosphate (pH 6.8), 0.1% sodium pyrophosphate, 5x Denhardt's solution, sonicated salmon sperm DNA (50 .mu.g/ml), 0.1% SDS, and 10% dextran sulfate at 42° C, with a 10 minute wash at 42°C in 0.2x SSC (sodium chloride/sodium citrate) followed by a 10 minute high-stringency wash consisting of 0.1x SSC containing EDTA at 55° C. Moderately stringent conditions may be identified as described by Sambrook et al., Molecular Cloning: A Laboratory Manual, New York: Cold Spring Harbor Press, 1989, and include the use of washing solution and hybridization conditions (e.g., temperature, ionic strength and % SDS) less stringent that those described above. An example of moderately stringent conditions is overnight incubation at 37°C in a solution comprising: 20% formamide, 5x SSC (150 mM NaCl, 15 mM trisodium citrate), 50 mM sodium phosphate (pH 7.6), 5x Denhardt's solution, 10% dextran sulfate, and 20 mg/ml denatured sheared salmon sperm DNA, followed by washing the filters in 1x SSC at about 37-50°C. The skilled artisan will recognize how to adjust the temperature, ionic strength, etc. as necessary to accommodate factors such as probe length and the like. The appropriate stringency for hybridizing nucleic acids depends on the length of the nucleic acids and the degree of complementation, variables well known in the art. The greater the degree of similarity or homology between two nucleotide sequences, the greater the value of Tm for hybrids of nucleic acids having those sequences. The relative stability (corresponding to higher Tm) of nucleic acid hybridizations decreases in the following order: RNA:RNA, DNA:RNA, DNA:DNA. Preferably a minimum length for a hybridizable nucleic acid is at least about 12 nucleotides; preferably at least about 16 nucleotides; and more preferably the length is at least about 24 nucleotides; and most preferably at least 36 nucleotides.

As used herein, "label" or "detectable-label" refers to a detectable label compound or composition which is conjugated directly or indirectly to an antibody, oligopeptide or other organic molecule so as to generate a "labeled" antibody, oligopeptide or other organic molecule. The label may be detectable by itself (e.g. radioisotope labels or fluorescent labels) or, in the case of an enzymatic label, may catalyze chemical alteration of a substrate compound or composition which is detectable.

As used herein, a "direct labels" refers an entity, which in its natural state, is readily visible, either to the naked eye, or with the aid of an optical filter and/or applied stimulation, e.g. ultraviolet light to promote fluorescence. Examples include, but are not limited to, colored labels, metallic sol particles, dye sol particles dyed latex or dyes encapsulated liposomes (described by U.S. Pat. No. 4,313,734, U.S. Pat. No. 4,373,932, WO 88/08534), EP-A 0 280 559, 0 281 327, U.S. Pat. No. 4,703,017). Other direct labels include a radionucleotide, radiopaque substances, a fluorescent moiety or a luminescent moiety.

As used herein, "indirect labels" refers to enzymes can also be used according to the present invention. Various types of enzyme linked immunoassays are well known in the art, for example, alkaline phosphatase and horseradish peroxidase, lysozyme, glucose-6-phosphate dehydrogenase, lactate dehydrogenase, urease, these and others have been discussed in detail by Eva Engvall in Enzyme Immunoassay ELISA and EMIT in Methods in Enzymology, 70:419-439 (1980) and in U.S. Pat. No. 4,857,453.

As used herein, the term "kit" refers to an article of manufacture comprising one or more containers and a label or package insert on or associated with the containers. The containers may contain a detectable-labeled antibody, detectable-labeled antibody fragments or detectable-labeled oligonucleotides.

Alternatively, the containers provide the means to obtain total RNA from a bodily sample, reverse transcribe the total RNA to obtain cDNA and subject the cDNA to a polymerase chain reaction using a set of primers wherein one or both primers are detectable-labeled. Additional containers of the kit may be included that contain, e.g., diluents and buffers, control antibodies, oligopeptides or small organic molecules. The label or package insert may provide a description of the composition as well as instructions for storage and for the intended in vitro or diagnostic use.

Furthermore, there may be employed conventional molecular biology, microbiology, and recombinant DNA techniques within the skill of the art. Such techniques are explained fully in the literature. See, *e.g.,* Sambrook, Fritsch & Maniatis, Molecular Cloning: A Laboratory Manual, Second Edition (1989) Cold Spring Harbor Laboratory Press, Cold Spring Harbor, New York (herein "Sambrook *et al.,* 1989"); DNA Cloning: A Practical Approach, Volumes I and II (D.N. Glover ed. 1985); Oligonucleotide Synthesis (M.J. Gait ed. 1984); Nucleic Acid Hybridization [B.D. Hames & S.J. Higgins eds. (1985)]; Transcription And Translation [B.D. Hames & S.J. Higgins, eds. (1984)]; Animal Cell Culture [R.I. Freshney, ed. (1986)]; Immobilized Cells And Enzymes [IRL Press, (1986)]; B. Perbal, A Practical Guide To Molecular Cloning (1984); F.M. Ausubel et al. (eds.), Current Protocols in Molecular Biology, John Wiley & Sons, Inc. (1994).

In general, RNAi technology uses synthetic or vector-generated double stranded RNA to induce the degradation of mRNA containing homologous sequences (McManus and Sharp, Nat Rev Genet 3:737, 2002). RNAi screening has been used to elucidate gene function in many organisms such as Caenorhabditis elegans (Simmer et al., PloS Biol 1:E12, 2003), Drosophila (Lum et al., Science 299:2039,2003) and mammalian cells (Aza-Blanc et al., Mol Cell 12:627,2003).

In the presentdisclosure, HCT116 colon cancer cells were used to screen siRNAs directed against 101 cancer-related genes, mostly comprising the kinase family members, and the docetaxel killing effects were quantified by high-resolution dose-response curves. Using this approach, applicants have shown that under-expression of 9 genes, including BubR1, Bub1, Mad2, Mps1 and GEFT Rac/CDC, can inhibit killing by docetaxel, whereas under-expressing 15 others, including Pim1, p21, TACC3 and Aurora-A, can potentiate docetaxel-induced cell death.

Thus, broadly the present disclosure extends to diagnostic methods and kits, as defined in the claims, that may be used to identify whether an individual who is either taking or considering taking docetaxel will be resistant or sensitive to the drug.

Applicants have observed drug resistance to docetaxel, whereby drug resistance is associated with the loss of an expanded set of checkpoint control genes including BubR1, Bub1, Mad2, Msp1 and GEFT Rac/CDC.

Increased docetaxel sensitivity corresponds with the loss of any one of several genes, including GBP-1, STK11, RXRA, Heel, SPF45, Raf1, RELB, MKK3, PRKCD, HSPA1A, BRAF35, Aurora-A, Pim-1, TACC3 and p21waf1/Cip1, and inhibitors against these genes may serve as a valuable adjunct to docetaxel therapy.

The mitotic checkpoint genes serve as a failsafe mechanism to delay anaphase by the inhibition of Cdc20-APC (anaphase-promoting complex), until the kinetochores of sister chromatids are properly attached to the mitotic spindle and aligned at the equator (Zhou et al., J Cell Sci 115:3547, 2002), thus allowing cells to reliably exit mitosis and enter additional cell cycles with a precise complement of DNA. In the presence of microtubule inhibitors, cells exiting from mitosis can no longer properly segregate their chromosomes and typically undergo apoptosis immediately or after several cell cycles (Taylor and McKeon, Cell 89:727, 1997). The combination of microtubule inhibitors with loss of mitotic checkpoint genes such as BubR1 allows cells to escape mitotic arrest without undergoing apoptosis and lead to chromosome instability (CIN) and aneuploidy (Shin et al., Cancer Cell 4:483, 2003). Recently, drug resistance data were generated when microtubule inhibitor paclitaxel was used to treat BubR1 and Mad2 down regulated MCF-7 cells (Sudo et al., Cancer Res 64:2502, 2004).

The Pim-1 gene encodes a serine/threonine kinase that belongs to a small family of related kinases. Pim-1 function has been linked to proliferation, differentiation, apoptosis, tumorigenesis, hypoxia, angiogenesis and mitosis (Wang et al., Biochim Biophys Acta 1593:45, 2002). The microscopic imaging data, in conjunction with AKT hypo-phosphorylation data and elevated caspase-3 activity measurements, demonstrate that Pim-1 down regulation enhanced docetaxel-induced apoptosis by inactivating AKT signaling, which mediates cell survival. Pim-1 and p21 have very similar characteristics in sensitizing HCT116 cells to docetaxel; consistent with reports showing that p21 is a phosphorylation substrate of Pim-1 (Wang et al., Biochim Biophys Acta 1593:45, 2002). Pim-1 down regulation induced some apoptosis in the absence of docetaxel but was more effective in the presence of drug, whereas down regulation of transforming acidic coiled-coil protein TACC3 did not induced cell death in the absence of docetaxel, indicating the RNAi alone had no effect on cell proliferation. These results suggest that depletion of different proteins can evoke different mechanisms for sensitizing cells to docetaxel.

Accordingly, activation of one or more of the genes described in Table I and II and protein levels of these genes may be utilized to select a therapeutic treatment strategy and to monitor effectiveness of the selected treatment strategy.

A method is disclosed that monitors the activation levels of one or more genes shown in Table I and II during the course of chemotherapy to assess and predict effectiveness of chemotherapy for the patient. Biopsies from tumors (*e.g.,* breast, colon, non-small cell lung, and gastric tumors) or short-term culture of tumor biopsies from undiagnosed cancer patients or patients currently undergoing treatment is processed in order to isolate DNA (Hafner et al., Arch Pathol Lab Med 127:1221, 2003; Rodriguez et al., Clin Cancer Res 10:5785, 2004), messenger RNA (Chang et al., Lancet. 362:340, 2003) and/or proteins (Espina et al., J Immunol Methods 290:121, 2004) to assess the levels of gene activation or protein levels of the genes described in Table I and II or a subset thereof. Gene transcription and protein expression profiling has utility in diagnosis, prediction of therapeutic responses to potential treatment strategies and may be a useful tool to monitor a patient's response to therapy. RNA may be isolated from tumor samples using any of the available methods commonly used in the art (Ullmann et al., J Biomol Screen 9:95, 2004; Badiee et al., BMC Biotechnol 3:23, 2003). One or more cells from test subjects are obtained and RNA is isolated from these cells. As examples, peripheral blood leukocytes (PBLs) cells may be obtained from the subject or it is also possible to obtain a cell sample and enrich the sample for a desired cell type. Cells may be isolated from a cell mixture using a variety of techniques, such as cell isolation using antibodies that bind to an particular epitope on the desired cell type. Where the desired cells are in a solid tissue, particular cells may be dissected, for example, by microdissection or by laser capture microdissection (LCM) (Bonner et al., Science 278:1481, 1997; Fend et al., Am J Path 154:61, 1999). RNA may be extracted from tissue or cell samples by a variety of methods, for example, guanidium thiocyanate lysis followed by CsCl centrifugation (Chirgwin et al., Biochemistry 18:5294, 1979). RNA from single cells may be obtained as described in methods for preparing cDNA libraries from single cells (Dulac, Curr Top Dev Biol. 36:245, 1998). The RNA sample can be further enriched for a particular species. For example, poly(A)+RNA may be isolated from an RNA sample. In particular, poly-T oligonucleotides may be immobilized on a solid support to serve as affinity ligands for mRNA. Kits for this purpose are commercially available, for example, the MessageMaker kit (Life Technologies, Grand Island, N.Y.). Enrichment may be accomplished, for example, by primer-specific cDNA synthesis, or multiple rounds of linear amplification based on cDNA synthesis and template-directed in vitro transcription (Wang et al., Proc Natl Acad. Sci USA 86:9717, 1989; Dulac *et al.,* supra).

A variety of amplification methods are suitable for use in the methods of the present invention, including, for example, PCR; ligase chain reaction (LCR) (Wu and Wallace, Genomics 4:560, 1989); self-sustained sequence replication (SSR) (Guatelli et al., Proc Natl Acad Sci USA 87:1874, 1990); nucleic acid based sequence amplification (NASBA) and transcription amplification (Kwoh et al., Proc Natl Acad Sci USA 86:1173, 1989). Methods for PCR technology are well known in the art (see, e.g., PCR Technology: Principles and Applications for DNA Amplification (ed. H. A. Erlich, Freeman Press, N.Y., N.Y., 1992); PCR Protocols: A Guide to Methods and Applications (eds. Innis, et al., Academic Press, San Diego, Calif., 1990).

The RNA may be labeled for detection by using any of the methods known to those persons skilled in the art. For example, using microarrays, such as those manufactured by Affymetrix. RNA is labeled and detected by hybridization using any of the methods commonly used, such as, direct RNA labeling with dyes such as Cyanine-3 or Cyanine-5 (Perkin Elmer MPS544001KT). Additional methods include direct cDNA labeling that is done by reverse transcription using nucleotides labeled with Cyanine-3 or Cyanine-5 (Badiee A 2003), fluorescein or Alexa dyes (Molecular Probes), or other fluorophores. Furthermore, indirect cDNA labeling such as FairPlay™/aminoallyl labeling (Stratagene Catalog number: 252002), 3DNA dendrimer labeling (Genisphere Inc) or enzymatic signal amplification (MICROMAX TSA, Perkin Elmer) may be utilized. Alternatively, RNA may be quantified by using RNA probes that are detected by enzymatic, fluorescence, radioactive or light emitting methods.

In one aspect of the present invention, RNA is obtained from the tumor sample and Taqman technology is employed in the detection of BubR1. Primers are generated to detect the specific RNA and amplified using reverse transcriptase. Detection of the specific fragment amplified by reverse transcription is accomplished by gel electrophoresis, polymer electrophoresis, direct DNA sequencing, light detection, loss of a fluorescence signal, enzymatic reaction, or detection by hybridization to a complementary RNA.

In another aspect of the present invention, Real Time PCR is utilized to detect BubR1 The mRNA obtained from a tumor sample is reverse translated into cDNA. Primers are generated to amplify the specific RNA using polymerase chain reaction technology. Detection of mRNA level is achieved by gel electrophoresis and ethidium bromide or CYBR green staining, or by measuring the fluorescence intensity from fluorophore-labeled sequence specific probes released by polymerase.

In a further aspect of the present invention, Northern blotting technology is utilized. RNA is obtained from the tumor sample and then separated by gel electrophoresis and transferred onto a membrane. BubR1 RNA abundance is determined by hybridization using probes labeled with radioactive isotope such as P³² or by enzymatic based chromogenic or luminescent assays.

In yet another aspect of the present invention, protein levels are used as a surrogate measure of transcript levels. Protein levels have been shown to correlate with suppression or elevation of the transcript levels. Thus, the present invention encompasses techniques to measure levels of proteins that correspond to the polypeptide products encoded by BubR1. Proteins are detected and used as markers of a predictive response to docetaxel. Methods of protein detection are well known in the art. For example, immunoassays are methodologies that employ antibodies to detect the expression of a target protein.

In a particular embodiment, an immunoassay is used to detect the protein products of BubR1. Additionally, antibodies against BubR1 may be used in a number of other detection methods. These detection methods include, but are not limited to, the following: Western blots, ELISA assays, sandwich ELISA. Alternatively, other assays not employing antibodies are contemplated and include approaches that utilize DNA oligonucleotides or polypeptides that recognize proteins encoded by BubR1 (*e.g*., aptamers). Mass spectrometric analysis of biological samples may also be used that determine the composition of polypeptides by determining the precise molecular weight of peptide fragments after proteolytic cleavage.

Suitable labels include enzymes, fluorophores (e.g., fluorescein isothiocyanate (FITC), phycoerythrin (PE), Texas red (TR), rhodamine, free or chelated lanthanide series salts, chromophores, radioisotopes, chelating agents, dyes, colloidal gold, latex particles, ligands (e.g., biotin), and chemiluminescent agents.

The radioactive label used may be isotopes such as 3H, 14C,32P, 35S, 36 Cl, 51 Cr, 57Co, 58Co,59Fe, 90Y, 125 I, 131I, and 186Re. Quantification of radioactivity levels may be performed through currently known and available counting procedures. Conversely, the use of radiopaque substances may be used.

Where the label is an enzyme, detection may be accomplished by any of the presently utilized colorimetric, spectrophotometric, fluorospectrophotometric, amperometric or gasometric techniques known in the art.

Also foreseen is the use of peptide binding agents such as antibodies or fragments of antibodies. Antibodies include polyclonal and monoclonal antibodies, prepared according to conventional methodology. Only a small portion of an antibody molecule, the paratope, is involved in the binding of the antibody to its epitope (see, in general, Clark, W. R. (1986) The Experimental Foundations of Modem Immunology Wiley & Sons, Inc., New York; Roitt, I. (1991) Essential Immunology, 7th Ed., Blackwell Scientific Publications, Oxford). The pFc' and Fc regions, for example, are effectors of the complement cascade but are not involved in antigen binding. An antibody from which the pFc' region has been enzymatically cleaved, or which has been produced without the pFc' region, designated an F(ab')2 fragment, retains both of the antigen binding sites of an intact antibody. Thus a detectable-lableled antibody fragment such as the F(ab')2 fragment may be utilised. Similarly, an antibody from which the Fc region has been enzymatically cleaved, or which has been produced without the Fc region, designated an Fab fragment, retains one of the antigen binding sites of an intact antibody molecule. Fab fragments consist of a covalently bound antibody light chain and a portion of the antibody heavy chain denoted Fd. The Fd fragments are the major determinant of antibody specificity (a single Fd fragment may be associated with up to ten different light chains without altering antibody specificity) and Fd fragments retain epitope-binding ability in isolation.

As is well-known in the art, within the antigen-binding portion of an antibody, there are complementarity determining regions (CDRs), which directly interact with the epitope of the antigen, and framework regions (FRs), which maintain the tertiary structure of the paratope (see, in general, Clark, 1986; Roitt, 1991). In both the heavy chain Fd fragment and the light chain of IgG immunoglobulins, there are four framework regions (FR1 through FR4) separated respectively by three complementarity determining regions (CDR1 through CDR3). The CDRs, and in particular the CDR3 regions, and more particularly the heavy chain CDR3, are largely responsible for antibody specificity. It is now well-established in the art that the non-CDR regions of a mammalian antibody may be replaced with similar regions of conspecific or heterospecific antibodies while retaining the epitopic specificity of the original antibody. This is most clearly manifested in the development and use of "humanized" antibodies in which non-human CDRs are covalently joined to human FR and/or Fc/pFc' regions to produce a functional antibody (refer to U.S. Pat. Nos. 4,816,567, 5,225,539, 5,585,089, 5,693,762 and 5,859,205).

Fully human monoclonal antibodies also can be prepared by immunizing mice transgenic for large portions of human immunoglobulin heavy and light chain loci. Following immunization of these mice (e.g., XenoMouse (Abgenix), HuMAb mice (Medarex/GenPharm)), monoclonal antibodies can be prepared according to standard hybridoma technology. These monoclonal antibodies will have human immunoglobulin amino acid sequences and therefore will not provoke human anti-mouse antibody (HAMA) responses when administered to humans.

Therefore, it is readily apparent to one of ordinary skill in the art, the present invention may use F(ab')2, Fab, Fv and Fd fragments; chimeric antibodies in which the Fc and/or FR and/or CDR1 and/or CDR2 and/or light chain CDR3 regions have been replaced by homologous human or non-human sequences; chimeric F(ab')2 fragment antibodies in which the FR and/or CDR1 and/or CDR2 and/or light chain CDR3 regions have been replaced by homologous human or non-human sequences; chimeric Fab fragment antibodies in which the FR and/or CDR1 and/or CDR2 and/or light chain CDR3 regions have been replaced by homologous human or non-human sequences; and chimeric Fd fragment antibodies in which the FR and/or CDR1 and/or CDR2 regions have been replaced by homologous human or non-human sequences. The present invention also includes so-called single chain antibodies.

The present invention may be better understood by reference to the following nonlimiting Examples, which are provided as exemplary of the invention. The following Examples are presented in order to more fully illustrate the preferred embodiments of the invention.

### EXAMPLES

### EXAMPLE 1: siRNA Screening

In order to identify genes that render cells more resistant or sensitive to docetaxel treatment, siRNAs against 101 genes were screened in HCT116 cells using a range of docetaxel concentrations for the generation of dose-response curves. Human colon cancer cell line HCT116 was obtained from ATCC and cultured in McCoy 5A supplemented with 100 units/ml penicillin, 100 µg/ml streptomycin, 4 mM L-glutamine and 10% fetal bovine serum at 37°C with 95% CO2 and 5% 02. The siRNA list was comprised of cancer-related genes, including those that were over expressed in docetaxel-resistant breast tumors as detected by gene expression profiling experiments (Chang et al., Lancet 362:362, 2003). Additional criteria were that the targets be amenable to drug development. The overall scheme is summarized as follows. On the first day, HCT116 cells were plated at 5,000 cells per well in a 96-well format and on the following day transfected with a pool of 3-4 siRNAs per gene. A representative sample of the siRNAs used is shown in Table I and II. Lipofectamine 2000 (Invitrogen) was used to transfect siRNAs (Dharmacon) into HCT 116 cells in 96 well plates. On the third day siRNA was removed and docetaxel was added at concentrations ranging from 0 to 40nM. On the sixth day cell viability was quantified using WST-1 assay. This is an assay that monitors the activity of mitochondrial dehydrogenases present in viable cells that results in the cleavage of tetrazolium salt WST-1 to form formazan. On the day of assay, media were taken off from 96 well plates. WST-1 reagent (Roche) was diluted 10 times in McCoy 5A medium and 100ul was added to each well. Plates were then incubated for 40 - 80 minutes at 37°C before reading on SpectroMax (Molecular Devices) at 450nm. The WST-1 values were clustered hierarchically by calculating the ratio of the experimental to the control siRNA and a total of 15 genes were characterized as conferring sensitivity, and 9 genes conferring resistance shown in Table III below. The sensitive and resistant populations could be further divided into additional groups: 1) where the siRNA effect was observed at lower concentrations of docetaxel (1-6 nM) to shift the IC50 or 2) where the major effect was observed at higher concentrations (> 6nM) with a smaller shift in IC50.

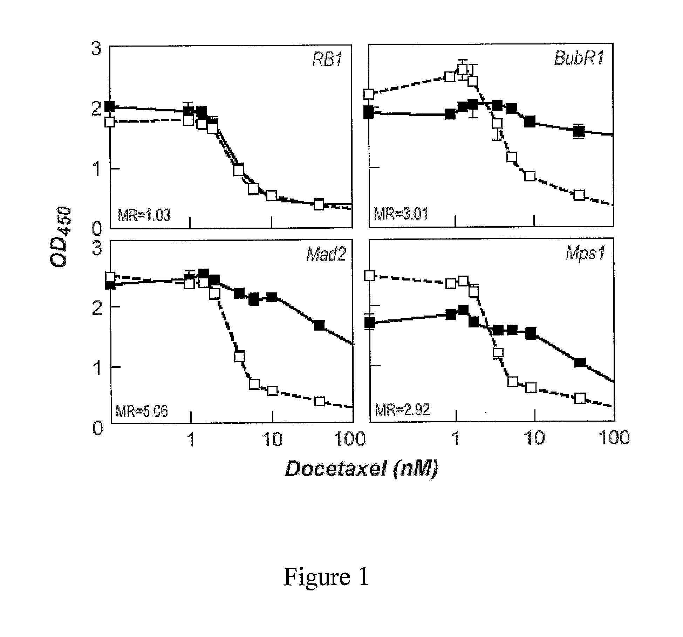

As compared to Mad2, BubR1 and Mps1 siRNAs, the siRNAs targeting Grb2, CDK6, sepharase, and calcium/calmodulin-dependent protein kinase II delta (CamKIID) showed more pronounced effects at lower docetaxel concentrations (1-6 nM), with a characteristic shift in the IC50. Overall, this subgroup showed weaker resistance than the mitotic checkpoint genes and the CamKIID siRNA generated the greatest level of protection, which was two-fold over control cells. These data demonstrate that loss of four mitotic checkpoint genes along with several other genes can increase docetaxel resistance to varying degrees. The data in Figure 1 show several characteristic dose-response curves for cells transfected with siRNAs followed by exposure to various concentrations of docetaxel. The siRNAs against four mitotic checkpoint genes, including BubR1, Bub1, Mad2, and Mps1 (data not shown for Bub1) as well as guanine nucleotide exchange factor (GEFT) for Rac/Cdc42 demonstrated significant docetaxel resistance with the effect being more pronounced at higher docetaxel concentrations (>6 nM). The greatest level of resistance was shown for Mad2 where cell viability increased five-fold over the control siRNA transfected cells. The majority of the siRNAs did not score in the screen and here we show human retinoblastoma 1 (RB1) as a representative example.

Taken together, these results show that siRNA screening can provide insights about how down-regulating gene expression sensitizes or protects cells from docetaxel treatment and, depending on the concentrations, evokes different mechanisms of action to modulate docetaxel-mediated cell death.

| **Table I: Sequences of siRNAs Conferring Increased Sensitivity to DocetaxeI:** | | | | |
|---|---|---|---|---|
| **Gene Name** | **siRNA sequence** | **SEQ ID NO** | **siRNA sequence** | **SEQ ID NO** |
| P21 (Waf1) | GCGAUGGAACUUCGACUUU | 1 | AGACCAUGUGGACCUGUCA | 3 |
| | UGGAACUUCGACUUUGUCA | 2 | | |
| Pim-1 | CCUUCGAAGAAAUCCAGAA* | 4 | UAUUCCUUUCGAGCAUGAC | 6 |
| | UGGUGUGUGGAGAUAUUCC | 5 | | |
| GBP-1 | CGAAAGGCAUGUACCAUAA | 7 | GAACAGGAGCAACUACUAA | 9 |
| | GAUACAGGCUGAAGAGAUU | 8 | | |
| RXRA | GCAAGGACCUGACCUACAC | 10 | GACCCUGUCACCAACAUUU | 12 |
| | GCAAGGACCGGAACGAGAA | 11 | | |
| SPF45 | CAAAUCCGCUGACUGAAAU | 13 | GACCCUAUGUUUCCUAAUG | 15 |
| | GAACAAGACAGACCGAGAU | 14 | | |
| Hec1 | GUGUAUUCGACAACUCUGU | 16 | GUACUCAGUUGCAGACAUU | 18 |
| | GAUUGCAAGAUUGGAACAA | 17 | GUAUCACAAAUUGGCUAGA | 19 |
| Raf1 | GCACGGAGAUGUUGCAGUA | 20 | GACAUGAAAUCCAACAAUA | 22 |
| | GCAAAGAACAUCAUCCAUA | 21 | GGAAUGAGCUUGCAUGACU | 23 |
| Aurora A | GAGAACUGCUACUUAUAUA | 24 | GAAGGUCGGAUGCAUGAUG | 26 |
| | GAAUAUGCACCACUUGGAA | 25 | GUAAAGGAAAGUUUGGUAA | 27 |
| TACC3 | GCACCUCGCUUCCCACAAG | 28 | GAACGAAGAGUCACUGAAG | 30 |
| | GAGCGGACCUGUAAAACUA | 29 | | |
| RelB | GCAGAAAGAGGACAUAUCA | 31 | GCCCGUCUAUGACAAGAAA | 33 |
| | GCAGCGAGCCAUUGCCUUU | 32 | | |
| PRKCD | GGACGUGGAUUGCAAACAA | 34 | GAAAGAACGCUUCAACAUC | 36 |
| | GCAUGAAUGUGCACCAUAA | 35 | | |
| BRAF35 | GGGCGUACCAGCAGUCUGA | 37 | GUCUGAAGCCUAUAAGAUG | 39 |
| | GCUCUGGGCUCAUGAACAC | 38 | | |
| HSPA1L | GAGAUCGACUCCCUGUUUG | 40 | GAUCAACGACGGAGACAAG | 42 |
| | UGGAGGAGUUCAAGAGAAA | 41 | | |
| STK11 | GAAGAAGGAAAUUCAACUA | 43 | GAAACAUCCUCCGGCUGAA | 45 |
| | GAGAAGCGUUUCCCAGUGU | 44 | | |
| MKK3 | GGUGGAGGCUGAUGACUUG | 46 | GGAGGGCCAUGUGAAGAUG | 48 |
| | GGAGAUUGCUGUGUCUAUC | 47 | GAUGUGAAGCCCUCCAAUG | 49 |
| *Individual sequences having demonstrated good knockdown | | | | |

| **Table II: Sequences of siRNAs Conferring Decreased Sensitivity to Docetaxel:** | | | | |
|---|---|---|---|---|
| **Gene Name** | **siRNA sequence** | **SEQ ID NO** | **siRNA sequence** | **SEQ ID NO** |
| BubR1 | GCAAUGAGCCUUUGGAUAU* | 50 | GGAAGAAGAUCUAGAUGUA | 52 |
| | GGAACAACCUCAUUCUAAA | 51 | | |
| Mad2 | GAAAUCGUGGCCGAGUUCU | 53 | GCCGAGUUCUUCUCAUUCG | 55 |
| | GUGGCAUAUAUCCAUCUGA | 54 | GGAACAACUGAAAGAUUGG | 56 |
| Mps1 | GUCGUUACAGUCAAGCAAU* | 57 | GAUGAACUAAGCUUGAAUA | 59 |
| | GCACGUGACUACUUUCAAA | 58 | GAGCAGUACCACUAGAAAU | 60 |
| GEFT | GAACACAGCCUGGAUAUGU | 61 | GGAUGAAGAUGAGCUGUAA | 63 |
| | GCACCGAGACUAUUUCUUG | 62 | GCAUGUGGCUCAGAUCUUG | 64 |
| Bub1 | GUACAACAGUGACCUCCA | 65 | GCUUGUGAUAAAGAGUCAA | 67 |
| | GAUGCUGGAUGUGUGAAUA | 66 | GAUCCACCAGAUGCUAUUG | 68 |
| hSeparase | GCUGUCAGAUAGUUGAUUU | 69 | GUGGACAGUUGUAAAUCUA | 71 |
| | GCCUACAGCUUCUAUAGUC | 70 | GAAGAUCGUUUCCUAUACA | 72 |
| CamKIID | GCACGAAAGCAAGAGAUUA | 73 | GCUAGAAUCUGCCGUCUUU | 75 |
| | GAAGAAACCAGAUGGAGUA | 74 | GAUCAAGGCUGGAGCUUAU | 76 |
| CDK6 | GAACAUGUCGAUCAAGACU | 77 | GAGUAGUGCAUCGCGAUCU | 79 |
| | GUUUGUAACAGAUAUCGAU | 78 | GUAACAGAUAUCGAUGAAC | 80 |
| GRB2 | GAACGAAGAAUGUGAUCAG | 81 | GGUACAAGGCAGAGCUUAA | 83 |
| | GUGGAUUAUCACAGAUCUA | 82 | GUGCCACAGCAGCCGACAU | 84 |
| | | | | |

| **Control Sequences:** | | | | |
|---|---|---|---|---|
| | GUCGACCAUUAUGUACCGGAU | 85 | GGAUUCGUAACGUGAUUAGCG | 86 |
| | | | | |

| **Sequences of siRNAs Not Affecting Docetaxel Response:** | | | | |
|---|---|---|---|---|
| RB1 | GAAACAGAAGAACCUGAUU | 87 | GUACAUCUCAGAAUCUUGA | 89 |
| | GAAAGGACAUGUGAACUUA | 88 | | |
| | | | | |
| * Individual sequences having demonstrated good knockdown | | | | |

**Table III: Genes Affecting Docetaxel Response**

| **siRNAs Conferring Increased Sensitivity to Docetaxel:** | | | | | | |
|---|---|---|---|---|---|---|
| **Gene Name** | **SEQ ID NO** | **Accession number** | **IC50 ratio** | **p(IC50 ratio)** | **Min ratio** | **p(Min ratio)** |
| P21 (Waf1) | 90 | NM_000389 | 0.70 | 0.0004 | 0.25 | 0.0000 |
| Pim-1 | 91 | NM_002648 | 0.80 | 0.0011 | 0.30 | 0.0000 |
| GBP-1 | 92 | NM_002053 | 0.59 | 0.0000 | 0.25 | 0.0000 |
| RXRA | 93 | NM_002957 | 0.64 | 0.0011 | 0.36 | 0.3098 |
| SPF45 | 94 | NM_032905 | 0.72 | 0.0000 | 0.47 | 0.0000 |
| Hec1 | 95 | NM_006101 | 0.64 | 0.0000 | 0.31 | 0.0121 |
| Raf1 | 96 | X03484 | 0.90 | 0.0059 | 0.30 | 0.0000 |
| Aurora A | 97 | AF008551 | 0.73 | 0.0000 | 1.37 | 0.0018 |
| TACC3 | 98 | NM_006342 | 0.29 | 0.0000 | 0.85 | 0.0077 |
| RelB | 99 | NM_006509 | 0.56 | 0.0000 | 0.77 | 0.0052 |
| PRKCD | 100 | NM_006254 | 0.46 | 0.0683 | 0.59 | 1.0000 |
| BRAF35 | 101 | NM_006339 | 0.44 | 0.0000 | 1.29 | 0.1361 |
| HSPA1L | 102 | NM_005345 | 0.60 | 0.0404 | 1.08 | 1.0000 |
| STK11 | 103 | NM_000455 | 0.94 | 0.4544 | 0.27 | 0.0000 |
| MKK3 | 104 | L36719 | 0.51 | 0.0000 | 0.82 | 0.0553 |
| | | | | | | |

| **siRNAs Conferring Increased Resistance to Docetaxel:** | | | | | | |
|---|---|---|---|---|---|---|
| **Gene Name** | **SEQ ID NO** | **Accession number** | **IC50 ratio** | **p(IC50 ratio)** | **Min ratio** | **p(Min ratio)** |
| BubR1 | 105 | AF046079 | 1.88 | 0.2207 | 3.01 | 0.5663^ |
| Mad2 | 106 | AJ000186 | 3.22 | 0.0076 | 5.06 | 1.0000^ |
| Mps1 | 107 | NM_003318 | 3.55 | 0.0008 | 2.92 | 1.0000^ |
| GEFT for Rac1/CDC42 | 108 | NM_133483 | 3.50 | 1.0000 | 2.42 | 1.0000^ |
| Bub1 | 109 | NM_004336 | 1.38 | 0.0007 | 2.45 | 0.0000 |
| hSeparase | 110 | NM_012291 | 1.99 | 0.0000 | 0.88 | 1.0000 |
| CamKIID | 111 | NM_001221 | 2.03 | 0.0016 | 1.29 | 1.0000 |
| CDK6 | 112 | NM_001259 | 1.99 | 0.0000 | 1.14 | 1.0000 |
| GRB2 | 113 | NM_002086 | 1.97 | 0.0000 | 1.16 | 1.0000 |
| | | | | | | |

| **siRNAs Having No Effect on the Response to Docetaxel:** | | | | | | |
|---|---|---|---|---|---|---|
| **Gene Name** | **SEQ ID NO** | **Accession number** | **IC50 ratio** | **p(IC50 ratio)** | **Min ratio** | **p(Min ratio)** |
| RB1 | 114 | NM_000321 | 0.87 | 0.0061 | 1.03 | 0.4863 |
| GAPDH | 115 | NM_002046 | nd | nd | nd | nd |
| RPS9 | 116 | BC071941 | nd | nd | nd | nd |
| | | | | | | |
| **Notes:** | | | | | | |
| Min ratio: The ratio of a siRNA over the control at the highest docetaxel dose | | | | | | |
| The smaller the p value is, the more significant the difference between sample and control | | | | | | |
| The p values marked with ^ are high because of poor curve fitting on the dose response curves, even though the differences are significant and reproducible | | | | | | |
| Nd=not determined | | | | | | |

### EXAMPLE 2: Cross Validation on a Subset of siRNAs that Confer Increased Docetaxel Resistance

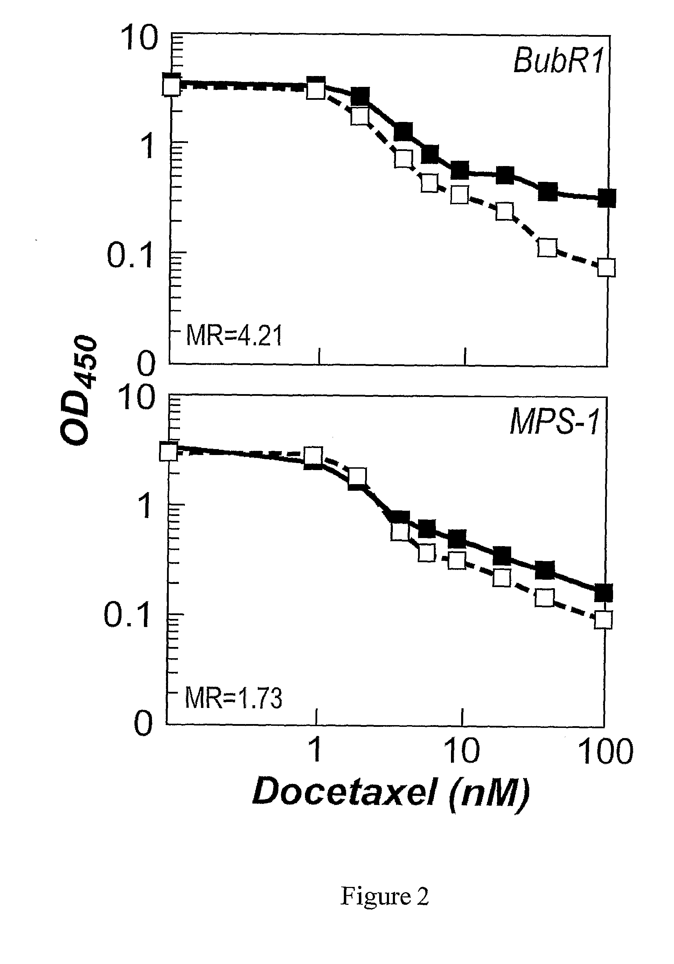

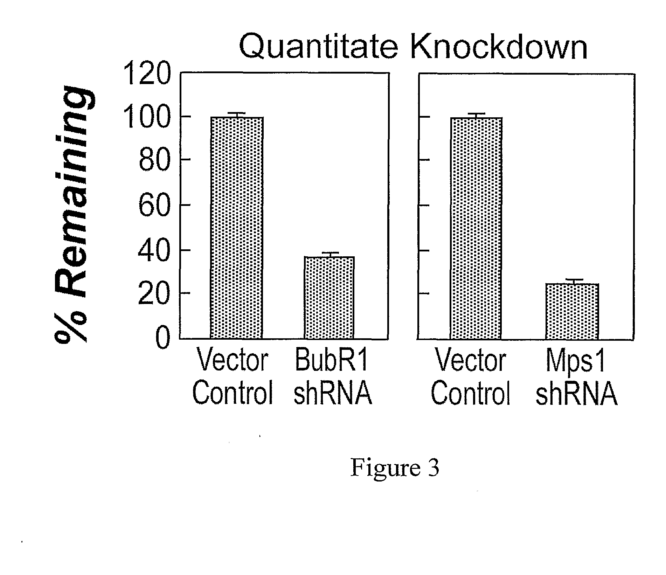

A potential drawback to siRNA screening approaches rests on the observation that siRNAs can anneal to transcripts with partial identity or initiate micro-RNA (miRNA) translational blocks, making it difficult to distinguish between target-specific effects and nonspecific (off-site) effects (Jackson et al., Nat Biotechnol 21:635, 2003). To confirm resistance using an independent approach with a second sequence, stable knock-down cells lines were generated for BubR1 and Mps1 by infecting HCT116 cells with a retroviral vector carrying DNA oligonucleotides encoding a short hairpin (sh)RNA. The shRNA contained a different sequence than the siRNA, targeting a distinct region of the open reading frame. The retroviral packaging cell line GP2-293 was obtained from Clontech. The cells were maintained in DMEM supplemented with 100 units/ml penicillin, 100 µg/ml streptomycin, 4 mM L-glutamine and 10% fetal bovine serum. Virus was generated by transiently transfecting GP2-293 cells. A total of 3.6 x 10⁶ cells were seeded in a 10 cm dish 24 hours prior to transfection. The medium was replaced 4 hours prior to transfection with DMEM containing 10% FBS minus antibiotics. The cells were transfected with 6 µg vector DNA, 6 µg of envelope plasmid VSV-G (Clontech) and 72 µl Lipfectamine-2000. The medium was replaced after 14-16 hours; the viral supernatant was harvested 24 hours later, filtered through a 0.45 µM filter and used to infect HCT116 cells at an M.O.I of 5 in the presence of 8 µg/ml polybrene. Forty-eight hours post-infection the cells were selected in 0.5 µg/ml puromycin for 7 days or until the background cells died off. The dose response curves generated with the BubR1 and Mps1 knock-down cell lines again showed enhanced resistance that was more pronounced at higher docetaxel concentrations (Figure 2), similar to the results obtained by siRNA transient transfections. To examine whether the shRNAs reduced mRNA levels we employed real-time PCR to detect endogenous transcripts and confirmed that the RNA levels were diminished in the BubR1 and Mps1 stable knock-down cell lines as compared to the vector control stable line (Figure 3).

Cells were lysed and RNA was extracted using RNAqueous -96 (Ambion). Taqman™ probes and forward and reverse primers were designed with Primer Express™ software (PE Applied Biosystems, UK). BLAST searches of the probe and primer sequences revealed no significant identity to other sequences other than the specific gene under test. For real time PCR, 20 µl of master TaqMan™ mix was made for each well: 4.825 µl Rnase-free water, 12.5 µl 2x Universal PCR master mix and 0.625 µl 40x MultiscribeTM and Rnase Inhibitor Mix (Applied BioSystems), 0.9 µl forward and reverse primers (100 µM), 0.25ul probe (100 µM). To each well, 5ul sample RNA (about 1ng/µl) was added. Plates were analyzed on a TaqMan™ ABI Prism 7700 Sequence Detector™ (Perkin-Elmer, UK). Cycling parameters were 48°C for 30 minutes, 95°C for 10 minutes, 40 cycles of 95°C for 15 seconds, and 60°C for 1 minute. Test gene mRNA values were extrapolated from the standard curve and presented as percent remaining.

### EXAMPLE 3: Downregulation of Mad2, BubR1 and Mps1 Protects Cells from Docetaxel-Induced Death by Escaping Mitotic Arrest, Coupled with the Generation of Aneuploidy

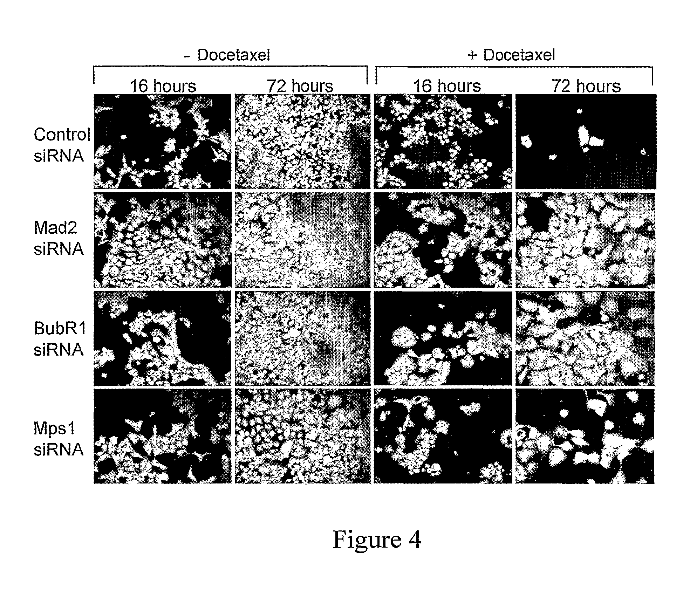

As determined by the WST-1 assay, loss of mitotic checkpoint genes in the presence of docetaxel correlated with increased cell viability to signify resistance (Figure 1). To further test the validity of WST-1 measurements, cytological experiments were performed to look at cell morphology and viability (Figure 4). The cells were tracked using confocal microscopy and Calcein-AM, a vital dye converted to green fluorescence by intracellular esterase serving as an indicator of metabolically active cells. Figure 4 demonstrates that 16 hours post docetaxel addition, the Mad2, BubR1 and, to a lesser extent, Mps1 siRNA transfected cells prematurely exited mitosis into an apparent interphase state coincident with a flattened cell morphology, whereas the majority of the control siRNA transfected cells were arrested in mitosis with a rounded up cell morphology. The flat cell phenotype has been previously described for other microtubule inhibitors and refers to the ability of cells to exit from mitotic arrest into an apparent interphase state without actually completing mitosis (Kung et al., Proc Natl Acad Sci 87:9553, 1990; Lanni and Jacks, Mol Cell Biol 18:1055, 1998). At 72 hours post treatment, the most striking observations for Mad2, BubR1 or Mps1 siRNA down-regulated cells were the presence of many more cells and their very large size, as compared to the siRNA control transfected cells that were virtually eliminated by docetaxel treatment. In the absence of docetaxel, mitotic checkpoint down-regulated cells looked similar to control cells at the two different time points and the increased number of cells at 72 hours suggested the cells were actively growing in the presence of the siRNA.

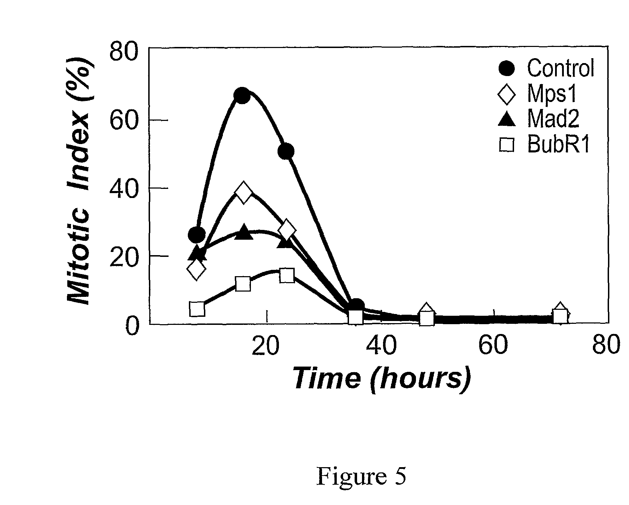

Mitotic checkpoint-impaired cells can bypass mitotic arrest and prematurely exit mitosis following treatment with microtubule inhibitors such as, colcemid, nocodazole and paclitaxel (Taylor and McKeon, Cell 89:727, 1997; Shin et al., Cancer Cell 4:483, 2003; Masuda et al., Am J Pathol 163:1109, 2003; Sudo et al., Cancer Res 64:2502, 2004). To determine if docetaxel treated HCT116 cells prematurely exit from mitosis following suppression of three specific checkpoint genes Mad2, BubR1 or Mps1, we measured mitotic indices of transfected cells. Accordingly, siRNA transfected Mad2, BubR1 or Mps1 cells were harvested without (0) treatment or at 8, 16, 24, 36, 48 and 72 hours after docetaxel treatment, then fixed and incubated with a polyclonal antibody that detects phosphorylation of histone H3 as an indication of mitosis (Mitotic Index Kit, Cellomics). A graphic representation for all time points are shown in Figure 5. The mitotic index peaked between 16 and 24 hours post treatment indicating that in all cases there was mitotic arrest. The mitotic index reached the highest value for cells transfected with the control siRNA, whereas cells transfected with Mad2 and BubR1 siRNA showed a significant decrease in mitotic index. The Mps1 siRNA effects were not as severe as Mad2 and BubR1. These results clearly show that mitotic checkpoint impaired cells can bypass mitotic arrest induced by docetaxel and that BubR1 siRNA was the most effective followed by Mad2 and Mps1.

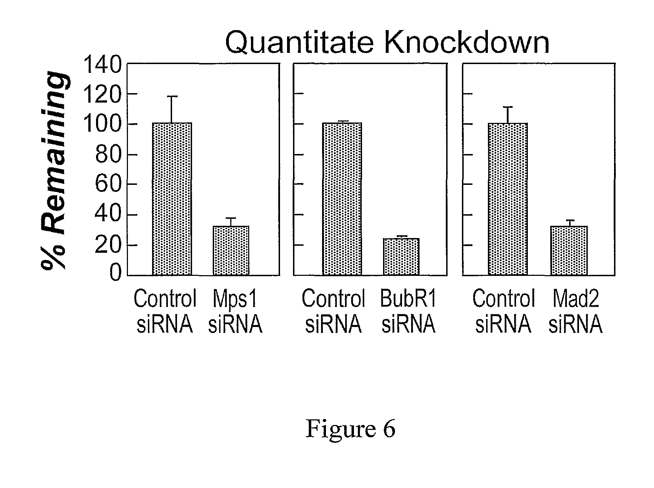

To rule out the possibility that changes in mitotic index were coincident with large differences in knockdown efficiencies, we employed real-time PCR to measure mitotic checkpoint transcript levels. The experiment shown in Figure 6 demonstrates that mRNA levels were diminished to comparable levels in Mad2, BubR1 and Mps1 siRNA transfected cells, suggesting that the mitotic index differences were more reflective of gene function.

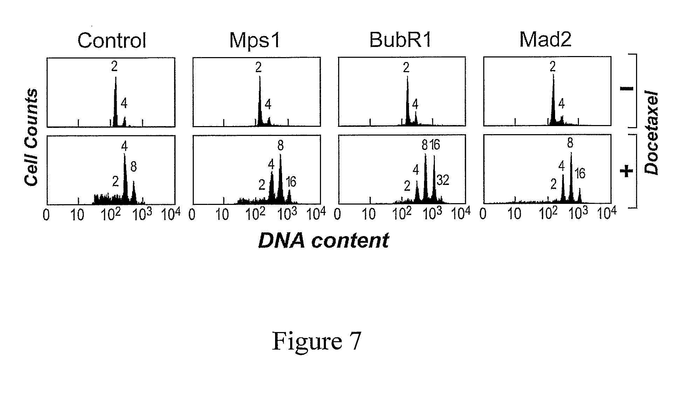

From the microscopy data, it appeared that the very large cells may harbor abnormal DNA content. In order to further investigate whether these mitotic checkpoint impaired cells were capable of overriding docetaxel-induced cell cycle arrest, and re-enter S-phase to generate aneuploid cells, we performed FACS analysis to measure DNA content. The, Mps1, BubR1 Mad2 and control siRNA transfected cells were grown in the absence or presence of docetaxel, fixed and stained with propidium iodide and analyzed by FACS. The data were collected at several different time points post docetaxel addition (data not shown) and histograms for the 72 hour time point are shown in Figure 7. At 72 hours post docetaxel addition, transfections with Mps1, BubR1, Mad2 and siRNAs caused an accumulation of cells with 8N, 16N and in the case of BubR1 32N DNA content. By just using the control siRNA, an 8N population of cells was detected in the presence of docetaxel, but the 8N numbers was far greater for the mitotic checkpoint down-regulated cells. At an earlier time point following docetaxel addition (data not shown), the majority of control cells were arrested at 4N, whereas cells transfected with Mps1, BubR1 and Mad2 siRNAs were advancing from 4N to 8N. These results indicate that knockdown of mitotic checkpoint genes allow cells to more rapidly accumulate abnormal DNA levels and cross a greater threshold of ploidy as compared to wild-type cells. Our results demonstrate roles for Mad2, BubR1 and Mps1 in regulating mitotic index and cell cycle progression with a previously uncharacterized microtubule inhibitor docetaxel.

### EXAMPLE 4: Formation of Docetaxel Resistant Colonies in BubR1 Downregulated Cells

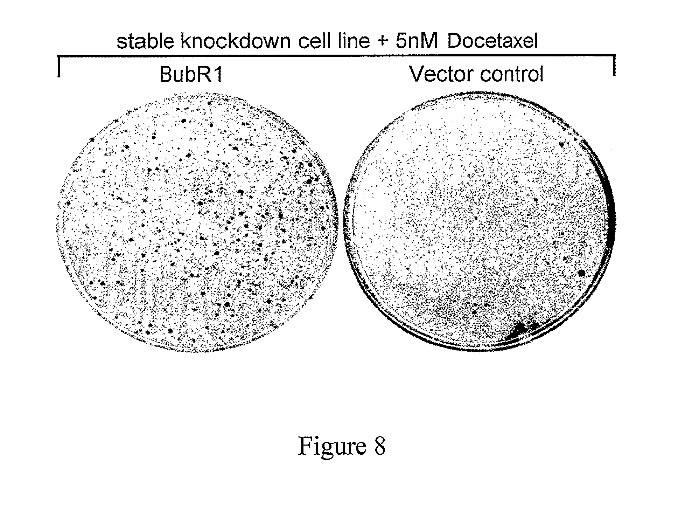

The apoptotic function of BubR1 appears to be important for the regulation of chromosome fidelity given that underexpression of BubR1 in combination with anti-microtubule agents was associated with aneuploidy, and restoration of BubR1 associated with an activated checkpoint and apoptosis of aneuploid cells (Shin et al., Cancer Cell 4:483, 2003). To determine if permanently downregulating mitotic checkpoint genes enables cells to grow with a greater proliferative capacity, the BubR1 and Mps1 stable knockdown lines (Figure 4) were tested for their ability to proliferate and establish colonies in the continuous presence of docetaxel. The Mad2 stable knockdown cell line could not be generated since impairment of Mad2 was lethal to cells (Michel et al., Proc Natl Acad Sci 101:4459, 2004). The BubR1, Mps1 and control shRNA stable knockdown cells lines were subjected to 5 nM docetaxel for 10 days and after that stained with crystal violet. Figure 8 shows that the BubR1 knockdown cell line produced many more large colonies as compared to the control cell line. The Mps1 knockdown cell line was not able to grow out colonies in the presence of 5 nM docetaxel. When the plates were incubated for as long as 17 days, colonies were observed on the control plates but were still fewer in number as compared to the BubR1 knockdown line. These results link low levels of BubR1, but not Mps1, with advanced cell growth in the presence of the chemotherapeutic drug docetaxel.

### EXAMPLE 5: Cross Validation of siRNA that Confers Increased Docetaxel Sensitivity

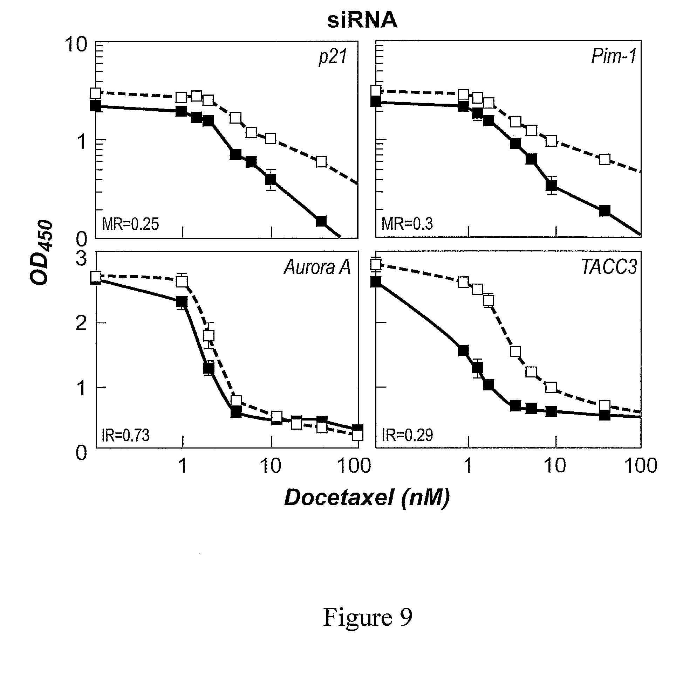

In addition to identifying siRNAs that protect cells from docetaxel-induced killing, the screen identified siRNAs that sensitize cells to killing. In Table III, Pim-1, p21^{waf1/Cip1}, GBP-1, RXRA, Heel, SPF45, Rafl represent a group of siRNAs that show pronounced killing at higher docetaxel concentrations (>6 nM) with just a small shift in IC₅₀. The siRNA dose-response curves for oncogenic serine/threonine kinase Pim-1 and cell cycle inhibitor p21^{waf1/Cip1} were very similar (Figure 9), which may reflect overlapping or cooperative roles within a signaling pathway; and previous reports have shown that p21^{wafl/Cip1} is a substrate for Pim-1 (Wang et al., Biochim Biophys Acta 1593:45, 2002). Of further interest were data on Pim-1 overexpression in prostate epithelial cells, which lead to mitotic checkpoint interference and genetic instability (Roh et al., Cancer Res 63:8079, 2003). These results are in alignment with Mad2, BubR1 and Mps1 underexpression data showing a comparable phenotype with opposing expression levels (see Figure 1). Another siRNA that conferred sensitivity to docetaxel was Hec1.

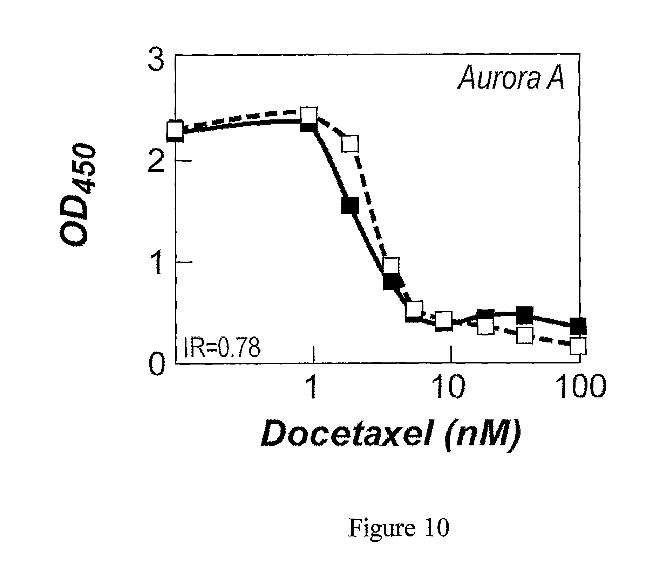

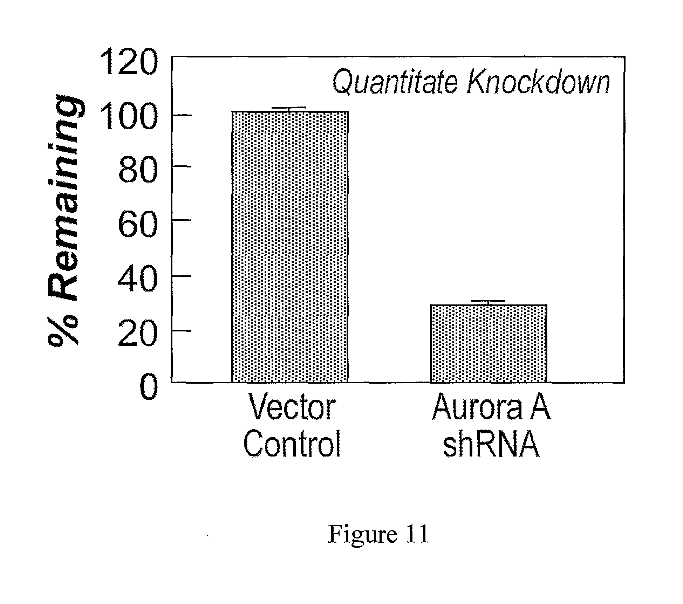

In Table III, TACC3, RELB, Aurora-A, PRKCD (PKC), HSPA1A, and BRAF35 represent siRNAs that increase sensitivity at lower docetaxel concentrations (1-6 nM) with a characteristic shift in IC50. Aurora-A is a serine/threonine kinase that functions at the onset of mitosis and has been implicated in centrosome maturation and spindle assembly (reviewed in Meraldi et al., Curr Opin Genet Dev 14:29, 2004). Of great interest were published data showing that Aurora-A over expression in HeLa cells protected cells from paclitaxel, in alignment with our results where under expression of Aurora-A enhanced docetaxel killing (Anand et al., Cancer Cell 3:51, 2003). Admittedly this was a small decrease in IC50 for Aurora-A representing a 25% decrease in survival; however, the results were precisely duplicated by using retroviral delivery of shRNA generating a stable knock-down of Aurora-A in HCT116 cells (compare Figures 9 to Figures 10 and 11).

### EXAMPLE 6: Pim1 Inhibits Proliferation of HCT116 cells in the Absence of Docetaxel

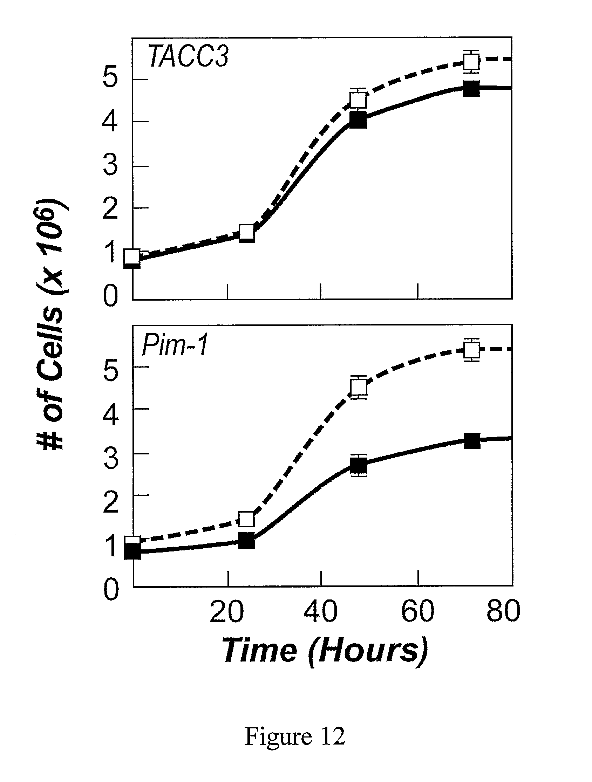

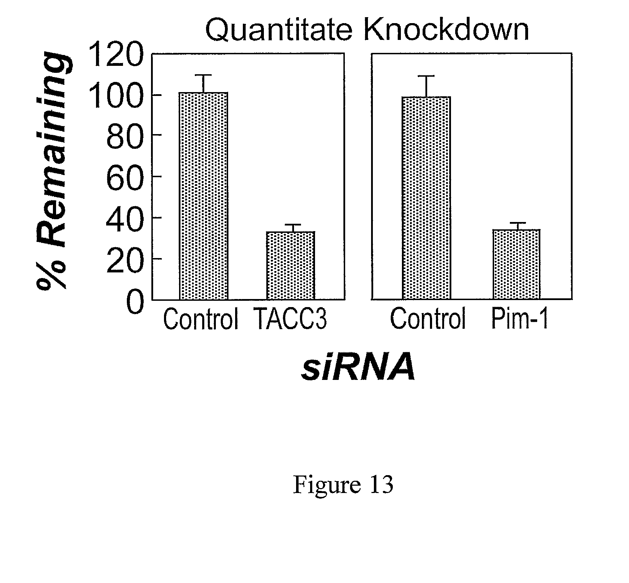

To further evaluate the positives from the screen, it was important to distinguish between siRNAs that affect cell viability in the absence of docetaxel, and those that act cooperatively with docetaxel to enhance killing. To distinguish between the possibilities we studied effects of siRNA transfection on proliferation using a trypan blue exclusion assay with cell counting to determine the number of viable cells in 6-well plates at 24, 48, 72 hours post-transfection. Our results show that Pim-1 siRNAs inhibit growth in HCT116 cells whereas TACC3 siRNAs have no effect on growth (Figure 12; knockdown quantitated in Figure 13), although both siRNAs enhance docetaxel-induced cell death (Figure 9).

### EXAMPLE 7: Validation of Subsets of Resistant and Sensitive Genes Using Live/Dead Assays

To visually compare siRNAs that confer docetaxel sensitivity and resistance, microscopic imaging is used to determine the ratio of live to dead cells where Calcein-AM is used to stain live cells green, and propidium iodide (PI) to stain the nucleus of dead cells red. Post docetaxel (72 hours) addition, siRNA transfected BubR1 cells had the greatest number of viable cells, whereas siRNA transfected Pim-1 and TACC3 had the greatest number of dead cells and the control cells were in between (data not shown). Although the BubR1 siRNA alone showed increased cell death by 72 hours, the cells ultimately become more resistant to docetaxel. Pim-1 siRNA alone induced cell death by 24 hours whereas the TACC3 siRNA did not cause death. These results demonstrate that BubR1 down regulation was associated with increased cell viability and drug resistance, whereas Pim-1 and TACC3 down regulation was associated with increased cell death and drug sensitivity. Both Pim-1 and TACC3 showed more killing in the presence of docetaxel but this assay could not distinguish between an additive effect and cooperative killing.

### EXAMPLE 8: Docetaxel-Induced Caspase-3 Activity was Diminished by BubR1 Down Regulation and Enhanced by Pim-1 Down Regulation

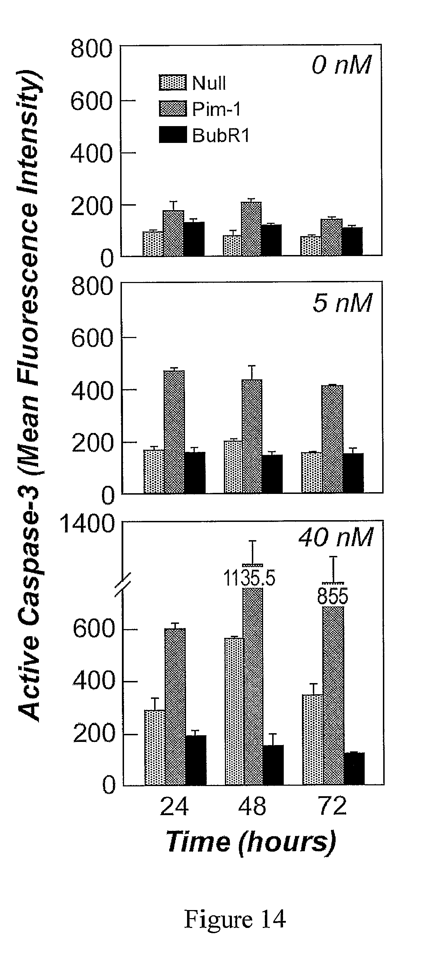

Docetaxel can cause cell death through induction of apoptosis (Kim et al., Int J Mol Med 11:799, 2003). To determine whether Pim-1 and BubR1 siRNAs could modulate a canonical apoptotic pathway in the absence and presence docetaxel, we studied activated caspase-3 levels in HCT116 cells (using a bioplex assay) (Figure 14). After 48 hours of 40 nM docetaxel, caspase-3 activity was induced in control cells and induction was further enhanced by down regulating Pim-1 and diminished by down regulating BubR1 (middle and lower panels). In the absence of docetaxel, down regulation of Pim-1 caused a slight increase in caspase-3 activity at 24 hours, but returned to control level by 48 and 72 hours (upper panel), indicating that other apoptotic machinery were activated to maintain the enhanced killing observed at 72 hours by WST-1 and LIVE/DEAD assays. Down regulation of BubR1 in the absence of docetaxel caused a very slight increase in caspase-3 activity, which is consistent with the observation that BubR1 knockdown initially caused cell death at 72 hours in the LIVE/DEAD assay. These results further implicate Pim-1 and BubR1 as effectors of docetaxel killing through the modulation of caspase-3 activity.

### EXAMPLE 9: Signaling Pathways Involved in Docetaxel-Induced Pim-1-Associated Sensitivity and BubR1-Associated Resistance

The activities attributed to Pim-1 strongly suggest that it plays a central role in blocking signaling events that lead to cell death while promoting those that foster cell survival. In an attempt to define the molecular basis for enhanced cell death associated with a Pim-1 knockdown, we examined the activation state of a key cellular pathway involved in cell survival, AKT. HCT116 were seeded at 4 X 10⁵ cells/well on 6-well plates and the next day transfected with siRNAs at 16 nM using Lipofectamine 2000 (Invitrogen). After 24 hours, cells were untreated or treated with 5 and 40 nM docetaxel. Cells were lysed 24, 48 and 72 hours after docetaxel addition with ice-cold lysis buffer (50 mM HEPES beffer (PH7.4), 1% NP40, 2.5 mM EDTA, 100 mM sodium fluoride, 10 mM sodium PP_{I}, protease inhibitor cocktail tablet (Roche), 2 mM sodium orthovanadate) and centrifuged at 12,000 g for 10 minutes. Lysates were then quantified for total protein levels using DC protein assay (BioRad). Active caspase-3 levels were determined using an active caspase-3 beadmates kit (Upstate) and the Luminex 100™ system with protocols suggested by the manufacture. Total and phosphorylated AKT levels were determined using a total AKT antibody bead kit and a phosphospecific AKT^{S473} antibody bead kit (Biosource) and the Luminex 100™ system with protocols suggested by manufacture.

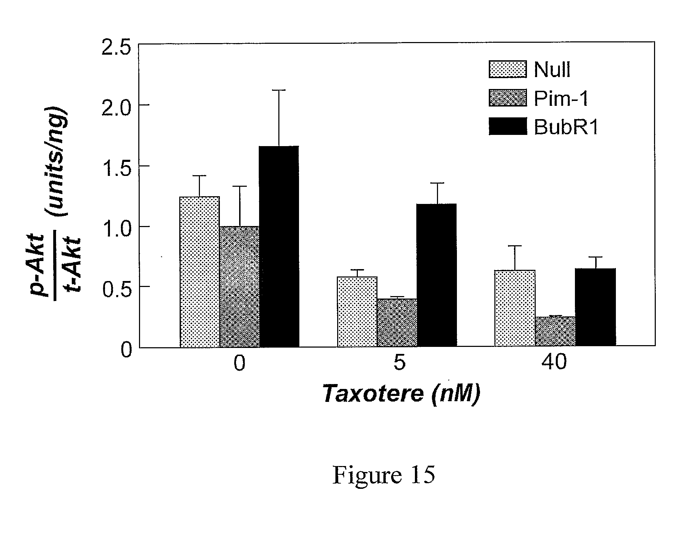

The experiment shown in Figure 15 demonstrates that Pim-1 downregulation decreased the baseline phosphorylation of AKT, as compared to the control and the effect was significantly accentuated with increasing concentrations of docetaxel. By comparison, BubR1 downregulation increased the baseline phosphorylation of AKT and the effect was accentuated at the lower but not higher concentration of docetaxel, indicating alternative-signaling pathways may be activated at higher doses.

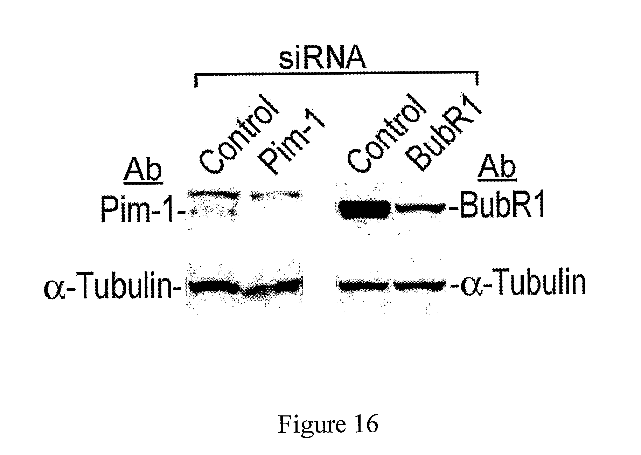

Applicants performed Western Blot analysis using antibodies against Pim-1 and BubR1 to measure protein levels at the 48 hour time point. Equal amounts of proteins were loaded into NuPAGE Novex Bis-Tris gels (Invitrogen). Antibodies against Pim-1 (Santa Cruz biotechnology), BubR1 (BD bioscience) and α-tubulin (Sigma) were used as suggested by the manufacture. Secondary antibodies were conjugated to HRP (BioRad) and detected by ECL Western Blot Detection System (Amhersham Biosciences) followed by exposure to Hyperfilm ECL (Amhersham Biosciences). The experiment shown in Figure 16 demonstrated that Pim-1 and BubR1 protein levels were diminished as compared to the control, showing that reduced protein levels corresponded with a functional change (Figure 15). These data suggest that downregulating Pim-1 protein levels can block the phosphorylation and activation of AKT in order to compel the cells towards apoptosis. Pim-1 appears to mediate cell survival by activating pro-survival pathways.
Anand S, Penrhyn-Lowe S, Venkitaraman AR. (2003) AURORA-A amplification overrides the mitotic spindle assembly checkpoint, inducing resistance to Taxol. Cancer Cell. 3:51-62.
Aza-Blanc P, Cooper CL, Wagner K, Batalov S, Deveraux QL, Cooke MP. (2003) Identification of modulators of TRAIL-induced apoptosis via RNAi-based phenotypic screening. Mol Cell. 12:627-37.
Chang JC, Wooten EC, Tsimelzon A, Hilsenbeck SG, Gutierrez MC, Elledge R, Mohsin S, Osborne CK, Chamness GC, Allred DC, O'Connell P. (2003) Gene expression profiling for the prediction of therapeutic response to docetaxel in patients with breast cancer. Lancet. 362:362-9.
Hong, WK (2002) The current status of docetaxel in solid tumors. Oncology 16:9-15.
Jackson AL, Bartz SR, Schelter J, Kobayashi SV, Burchard J, Mao M, Li B, Cavet G, Linsley PS. (2003) Expression profiling reveals off-target gene regulation by RNAi. Nat Biotechnol. 21:635-7.
Katsumata N. (2003) Docetaxel: an alternative taxane in ovarian cancer. Br J Cancer. 89 Suppl 3:S9-S15.
Kim R, Tanabe K, Uchida Y, Emi M, Toge T. (2003) Effect of Bcl-2 antisense oligonucleotide on drug-sensitivity in association with apoptosis in undifferentiated thyroid carcinoma. Int J Mol Med. 11:799-804.
Kolfschoten GM, Hulscher TM, Duyndam MC, Pinedo HM, Boven E. (2002) Variation in the kinetics of caspase-3 activation, Bcl-2 phosphorylation and apoptotic morphology in unselected human ovarian cancer cell lines as a response to docetaxel. Biochem Pharmacol. 63:733-43.
Kung AL, Sherwood SW, Schimke RT. (1990) Cell line-specific differences in the control of cell cycle progression in the absence of mitosis. Proc Natl Acad Sci USA. 87:9553-7.
Lanni JS, Jacks T. (1998) Characterization of the p53-dependent postmitotic checkpoint following spindle disruption. Mol Cell Biol. 18:1055-64.
Lee LF, Li G, Templeton DJ, Ting JP. (1998) Paclitaxel (Taxol)-induced gene expression and cell death are both mediated by the activation of c-Jun NH2-terminal kinase (JNK/SAPK). J Biol Chem. 273:28253-60.
Li Y, Dowbenko D, Lasky LA. (2002) AKT/PKB phosphorylation of p21Cip/WAF1 enhances protein stability of p21Cip/WAF1 and promotes cell survival. J Biol Chem. 277:11352-61.
Lum L, Yao S, Mozer B, Rovescalli A, Von Kessler D, Nirenberg M, Beachy PA. (2003) Identification of Hedgehog pathway components by RNAi in Drosophila cultured cells. Science. 299:2039-45.
Masuda A, Maeno K, Nakagawa T, Saito H, Takahashi T. (2003) Association between mitotic spindle checkpoint impairment and susceptibility to the induction of apoptosis by anti-microtubule agents in human lung cancers. Am J Pathol. 163:1109-16.
McManus MT, Sharp PA. (2002) Gene silencing in mammals by small interfering RNAs. Nat Rev Genet. 3:737-47.
Meraldi P, Honda R, Nigg EA. (2004) Aurora kinases link chromosome segregation and cell division to cancer susceptibility. Curr Opin Genet Dev. 14:29-36.
Michel L, Diaz-Rodriguez E, Narayan G, Hernando E, Murty VV, Benezra R. (2004) Complete loss of the tumor suppressor MAD2 causes premature cyclin B degradation and mitotic failure in human somatic cells. Proc Natl Acad Sci USA. 101:4459-64.
Ojima, I. and Geney, R. 109881 Aventis (2004) Curr Opin Investig Drugs 4:737-40.
Ringel I, Horwitz SB. (1991) Studies with RP 56976 (taxotere): a semisynthetic analogue of taxol. J Natl Cancer Inst. 83:288-91.
Roh M, Gary B, Song C, Said-Al-Naief N, Tousson A, Kraft A, Eltoum IE, Abdulkadir SA. (2003) Overexpression of the oncogenic kinase Pim-1 leads to genomic instability. Cancer Res. 63 :8079-84.
Shin HJ, Baek KH, Jeon AH, Park MT, Lee SJ, Kang CM, Lee HS, Yoo SH, Chung DH, Sung YC, McKeon F, Lee CW. (2003) Dual roles of human BubR1, a mitotic checkpoint kinase, in the monitoring of chromosomal instability. Cancer Cell. 4:483-97.
Simmer F, Moorman C, Van Der Linden AM, Kuijk E, Van Den Berghe PV, Kamath R, Fraser AG, Ahringer J, Plasterk RH. (2003) Genome-Wide RNAi of C. elegans Using the Hypersensitive rrf-3 Strain Reveals Novel Gene Functions. PLoS Biol. 1:E12.
Sudo T, Nitta M, Saya H, Ueno NT. (2004) Dependence of paclitaxel sensitivity on a functional spindle assembly checkpoint. Cancer Res. 64:2502-8.
Tanabe K, Kim R, Inoue H, Emi M, Uchida Y, Toge T. (2003) Antisense Bcl-2 and HER-2 oligonucleotide treatment of breast cancer cells enhances their sensitivity to anticancer drugs. Int J Oncol. 22:875-81.
Taylor SS, McKeon F. (1997) Kinetochore localization of murine Bub1 is required for normal mitotic timing and checkpoint response to spindle damage.Cell. 89:727-35.
Wang TH, Wang HS, Soong YK (2000) Paclitaxel-induced cell death: where the cell cycle and apoptosis come together. Cancer. 88:2619-28.
Wang Z, Bhattacharya N, Mixter PF, Wei W, Sedivy J, Magnuson NS. (2002) Phosphorylation of the cell cycle inhibitor p21Cipl/WAF1 by Pim-1 kinase. Biochim Biophys Acta. 1593:45-55.
Zhou J, Yao J, Joshi HC. (2002) Attachment and tension in the spindle assembly checkpoint. J Cell Sci. 115:3547-55.

## Claims

1. A method for predicting or monitoring a cancer patient's response to a molecule of the taxoid family, comprising the steps of:
a) measuring the level of BubR1 in a test sample from a cancerous area of said patient;
b) measuring the level of BubR1 in a control sample;
c) comparing the measured levels of BubR1 in the test sample and the control sample; wherein a decrease in the level of BubR1 measured in the test sample as compared to the control sample indicates an increased resistance to a molecule of the taxoid family.

2. The method of Claim 1 wherein said molecule of the taxoid family is paclitaxel, docetaxel. XRP9881 or XRP6258.

3. The method of Claim 1 wherein said level of BubR1 is measured by mRNA or protein.

4. The method of Claim 3 wherein said mRNA is measured by *in situ* hybridization, reverse-transcriptase polymerase chain reaction, nucleic acid hybridization, electrophoresis, Northern blotting or mass spectrometry.

5. The method of Claim 3 wherein said protein is measured by immunoassay, Western blots, ELISA or mass spectrometry.

6. Use of a kit for predicting or monitoring a cancer patient's response to a molecule of the taxoid family, said kit comprising:
a) an RNA recovery solution that isolates total RNA from a bodily sample;
b) an enzyme that reverse transcribes the total RNA to obtain cDNA; and
c) a set of primers that amplifies the DNA by a polymerase chain reaction;
wherein one or both primers are detectably-labeled and both primers will amplify a nucleotide sequence of BubR1.

7. The use of claim 6, wherein the kit further comprises detectable-labeled antibodies, detectable-labeled antibody fragments, or detectable-labeled oligonucleotides comprising nucleic acids capable of hybridizing under stringent conditions to BubR1, and wherein the detectable label is an enzyme, a radioactive isotope, a chemical which fluoresces, a chemiluminescent molecule, a radiopaque substance, a liposome, or a haptenic molecule.

8. A method for predicting or monitoring a cancer patient's response to a molecule of the taxoid family, comprising the steps of:
a) measuring the level of BubR1 in a test sample from a cancerous area of said patient;
b) measuring the level of one or more reference genetic markers in the test sample;
c) comparing the measured levels of BubR1 and said one or more reference genetic markers;
wherein a decrease in the level of BubR1 as compared to the level of said one or more reference genetic markers indicates an increased resistance to a molecule of the taxoid family.

9. The method of Claim 8 wherein said molecule of the taxoid family is paclitaxel, docetaxel, XRP9881 or XRP6258.

10. The method of Claim 8 wherein said level of one or more genetic markers is measured by mRNA or protein.

11. The method of Claim 10 wherein said mRNA is measured by *in situ* hybridization, reverse-transcriptase polymerase chain reaction, nucleic acid hybridization, electrophoresis, Northern blotting or mass spectrometry.

12. The method of Claim 10 wherein said protein is measured by immunoassay, Western blots, ELISA or mass spectrometry.

13. Use of a kit for predicting or monitoring a cancer patient's response to a molecule of the taxoid family, said kit comprising:
a) an RNA recovery solution that isolates total RNA from a bodily sample;
b) an enzyme that reverse transcribes the total RNA to obtain cDNA;
c) a first set of primers that will amplify the cDNA associated with BubR1 by a polymerase chain reaction; and
d) a second set of primers that will amplify the cDNA associated with a reference marker by a polymerase chain reaction;
wherein one or more primers are detectable-labeled.

14. The use of claim 13, wherein the kit further comprises detectable-labeled antibodies, detectable-labeled antibody fragments or detectable-labeled oligonucleotides comprising nucleic acids capable of hybridizing under stringent conditions to BubRl, and wherein the detectable label is an enzyme, a radioactive isotope, a chemical which fluoresces, a chemiluminescent molecule, a radiopaque substance, a liposome, or a haptenic molecule.

## Patentansprüche

1. Verfahren zur Vorhersage oder Kontrolle der Reaktion eines Krebspatienten auf ein Molekül der Taxoidfamilie, welches die folgenden Schritte umfasst:
a) Messen des BubR1-Spiegels in einer Testprobe aus einer krebsbefallenen Region des Patienten,
b) Messen des BubR1-Spiegels in einer Kontrollprobe,
c) Vergleichen der gemessenen BubR1-Spiegel in der Testprobe und der Kontrollprobe,
wobei eine Abnahme des in der Testprobe gemessenen BubR1-Spiegels im Vergleich zur Kontrollprobe auf eine erhöhte Resistenz gegenüber einem Molekül der Taxoidfamilie hindeutet.

2. Verfahren nach Anspruch 1, wobei es sich bei dem Molekül der Taxoidfamilie um Paclitaxel, Docetaxel, XRP9881 oder XRP6258 handelt.

3. Verfahren nach Anspruch 1, wobei der BubR1-Spiegel mittels mRNA oder Protein gemessen wird.

4. Verfahren nach Anspruch 3, wobei die mRNA durch in-situ-Hybridisierung, reverse Transkriptase-Polymerase-Kettenreaktion, Nukleinsäurehybridisierung, Elektrophorese, Northern-Blotting oder Massenspektrometrie gemessen wird.

5. Verfahren nach Anspruch 3, wobei das Protein durch Immunassay, Western-Blots, ELISA oder Massenspektrometrie gemessen wird.

6. Verwendung eines Kits zur Vorhersage oder Kontrolle der Reaktion eines Krebspatienten auf ein Molekül der Taxoidfamilie, wobei das Kit Folgendes umfasst:
a) eine Lösung zur Gewinnung von RNA, die die Gesamt-RNA aus einer Körperprobe isoliert,
b) ein Enzym, das die Gesamt-RNA unter Erhalt von cDNA revers transkribiert und
c) ein Primer-Set, das die cDNA durch eine Polymerase-Kettenreaktion amplifiziert,
wobei einer oder beide Primer nachweisbar markiert sind und beide Primer eine Nukleotidsequenz von BubR1 amplifizieren.

7. Verwendung nach Anspruch 6, wobei das Kit weiterhin nachweisbar markierte Antikörper, nachweisbar markierte Antikörperfragmente oder nachweisbar markierte Oligonukleotide, umfassend unter stringenten Bedingungen zur Hybridisierung mit BubR1 fähige Nukleinsäuren, umfasst, und wobei es sich bei dem nachweisbaren Marker um ein Enzym, ein radioaktives Isotop, eine fluoreszierende Chemikalie, ein chemiluminsezentes Molekül, eine röntgenopake Substanz, ein Liposom oder ein haptenisches Molekül handelt.

8. Verfahren zur Vorhersage oder Kontrolle der Reaktion eines Krebspatienten auf ein Molekül der Taxoidfamilie, welches die folgenden Schritte umfasst:
a) Messen des BubR1-Spiegels in einer Testprobe aus einer krebsbefallenen Region des Patienten,
b) Messen des Spiegels eines oder mehrerer genetischer Vergleichsmarker in der Testprobe,
c) Vergleichen des gemessenen BubR1-Spiegels und des Spiegels des einen oder der mehreren genetischen Vergleichsmarker,
wobei eine Abnahme des BubR1-Spiegels im Vergleich zu dem Spiegel des einen oder der mehreren genetischen Vergleichsmarker auf eine erhöhte Resistenz gegenüber einem Molekül aus der Taxoidfamilie hindeutet.

9. Verfahren nach Anspruch 8, wobei es sich bei dem Molekül der Taxoidfamilie um Paclitaxel, Docetaxel, XRP9881 oder XRP6258 handelt.

10. Verfahren nach Anspruch 8, wobei der Spiegel des einen oder der mehreren genetischen Marker mittels mRNA oder Protein gemessen wird.

11. Verfahren nach Anspruch 10, wobei die mRNA durch in-situ-Hybridisierung, reverse Transkriptase-Polymerase-Kettenreaktion, Nukleinsäurehybridisierung, Elektrophorese, Northern-Blotting oder Massenspektrometrie gemessen wird.

12. Verfahren nach Anspruch 10, wobei das Protein durch Immunassay, Western-Blots, ELISA oder Massenspektrometrie gemessen wird.

13. Verwendung eines Kits zur Vorhersage oder Kontrolle der Reaktion eines Krebspatienten auf ein Molekül der Taxoidfamilie, wobei das Kit Folgendes umfasst:
a) eine Lösung zur Gewinnung von RNA, die die Gesamt-RNA aus einer Körperprobe isoliert,
b) ein Enzym, das die Gesamt-RNA unter Erhalt von cDNA revers transkribiert,
c) ein erstes Primer-Set, das die mit BubR1 assoziierte cDNA durch eine Polymerase-Kettenreaktion amplifiziert und
d) ein zweites Primer-Set, das die mit einem Vergleichsmarker assoziierte cDNA durch Polymerase-Kettenreaktion amplifiziert,
wobei ein oder mehrere Primer nachweisbar markiert sind.

14. Verwendung nach Anspruch 13, wobei das Kit weiterhin nachweisbar markierte Antikörper, nachweisbar markierte Antikörperfragmente oder nachweisbar markierte Oligonukleotide, umfassend unter stringenten Bedingungen zur Hybridisierung mit BubR1 fähige Nukleinsäuren, umfasst, und wobei es sich bei dem nachweisbaren Marker um ein Enzym, ein radioaktives Isotop, eine fluoreszierende Chemikalie, ein chemiluminsezentes Molekül, eine röntgenopake Substanz, ein Liposom oder ein haptenisches Molekül handelt.

## Revendications

1. Procédé pour prédire ou surveiller une réponse d'un patient cancéreux à une molécule de la famille des taxoides, comprenant les étapes de :
a) mesure du taux de BubR1 dans un échantillon d'essai d'une zone cancéreuse dudit patient ;
b) mesure du taux de BubR1 dans un échantillon témoin ;
c) comparaison des taux mesurés de BubR1 dans l'échantillon d'essai et l'échantillon témoin
dans lequel une diminution du taux de BubR1 mesuré dans l'échantillon d'essai par rapport à l'échantillon témoin indique une résistance accrue à une molécule de la famille des taxoïdes.

2. Procédé de la revendication 1 dans lequel ladite molécule de la famille des taxoïdes est le paclitaxel, le docétaxel, XRP9881 ou XRP6258.

3. Procédé de la revendication 1 dans lequel ledit taux de BubR1 est mesuré par ARNm ou protéine.

4. Procédé de la revendication 3 dans lequel ledit ARNm est mesuré par hybridation *in situ,* réaction de polymérase en chaîne par transcriptase inverse, hybridation d'acide nucléique, électrophorèse, transfert Northern ou spectrométrie de masse.

5. Procédé de la revendication 3 dans lequel ladite protéine est mesurée par immunoessai, transfert Western, ELISA ou spectrométrie de masse.

6. Utilisation d'un kit pour prédire ou surveiller la réponse d'un patient cancéreux à une molécule de la famille des taxoides, ledit kit comprenant :
a) une solution de récupération d'ARN qui isole l'ARN total à partir d'un échantillon corporel ;
b) une enzyme qui transcrit de façon inverse l'ARN total pour obtenir un ADNc ; et
c) un ensemble d'amorces qui amplifie l'ADNc par une réaction de polymérase en chaîne ;
dans laquelle une ou les deux amorces sont marquées de façon détectable et les deux amorces amplifient une séquence nucléotidique de BubR1.

7. Utilisation de la revendication 6, dans laquelle le kit comprend en outre des anticorps marqués de façon détectable, des fragments d'anticorps marqués de façon détectable, ou des oligonucléotides marqués de façon détectable comprenant des acides nucléiques capables de s'hybrider dans des conditions stringentes à BubR1, et le marqueur détectable étant une enzyme, un isotope radioactif, une substance chimique qui émet une fluorescence, une molécule chimioluminescente, une substance radio-opaque, un liposome, ou une molécule hapténique.

8. Procédé pour prédire ou surveiller une réponse d'un patient cancéreux à une molécule de la famille des taxoïdes, comprenant les étapes de :
a) mesure du taux de BubR1 dans un échantillon d'essai d'une zone cancéreuse dudit patient ;
b) mesure du taux d'un ou plusieurs marqueurs génétiques de référence dans l'échantillon d'essai ;
c) comparaison des taux mesurés de BubR1 et desdits un ou plusieurs marqueurs génétiques de référence ;
dans lequel une diminution du taux de BubR1 par rapport au taux desdits un ou plusieurs marqueurs génétiques de référence indique une résistance accrue à une molécule de la famille des taxoïdes

9. Procédé de la revendication 8 dans lequel ladite molécule de la famille des taxoïdes est le paclitaxel, le docétaxel, XRP9881 ou XRP6258.

10. Procédé de la revendication 8 dans lequel ledit taux d'un ou plusieurs marqueurs génétiques est mesuré par ARNm ou protéine.

11. Procédé de la revendication 10 dans lequel ledit ARNm est mesuré par hybridation *in situ,* réaction de polymérase en chaîne par transcriptase inverse, hybridation d'acide nucléique, électrophorèse, transfert Northern ou spectrométrie de masse.

12. Procédé de la revendication 10 dans lequel ladite protéine est mesurée par immunoessai, transfert Western, ELISA ou spectrométrie de masse.

13. Utilisation d'un kit pour prédire ou surveiller la réponse d'un patient cancéreux à une molécule de la famille des taxoïdes, ledit kit comprenant :
a) une solution de récupération d'ARN qui isole l'ARN total à partir d'un échantillon corporel ;
b) une enzyme qui transcrit de façon inverse l'ARN total pour obtenir un ADNc ; et
c) un premier ensemble d'amorces qui amplifie l'ADNc associé à BubR1 une réaction de polymérase en chaîne ;
d) un deuxième ensemble d'amorces qui amplifie l'ADNc associé à un marqueur de référence par une réaction de polymérase en chaîne ;
dans laquelle une ou plusieurs amorces sont marquées de façon détectable.

14. Utilisation de la revendication 13, dans laquelle le kit comprend en outre des anticorps marqués de façon détectable, des fragments d'anticorps marqués de façon détectable ou des oligonucléotides marqués de façon détectable comprenant des acides nucléiques capables de s'hybrider dans des conditions stringentes avec BubR1, et dans laquelle le marqueur détectable est une enzyme, un isotope radioactif, une substance chimique qui émet une fluorescence, une molécule chimioluminescente, une substance radio-opaque, un liposome, ou une molécule hapténique.
